# FASCICULE DE BREVET EUROPEEN

(11) **EP 2 613 717 B1**
(45) Date de publication et mention de la délivrance du brevet: **05.11.2014**
(21) Numéro de dépôt: 11773060.6
(22) Date de dépôt: 09.09.2011
(51) Int. Cl.: A61B 17/128, A61B 17/115, A61B 17/122, A61B 17/00

(54) **SYSTÈME CHIRURGICAL POUR RÉALISER UNE ANASTOMOSE ENTRE DEUX CONDUITS CREUX D'UN PATIENT, NOTAMMENT ENTRE LA VESSIE ET L'URÈTRE**
CHIRURGISCHES BEHANDLUNGSSYSTEM ZUR DURCHFÜHRUNG EINER ANASTOMOSE ZWISCHEN ZWEI HOHLKANÄLEN BEI EINEM PATIENTEN, IM BESONDEREN ZWISCHEN BLASE UND HARNRÖHRE
SURGICAL TREATMENT SYSTEM FOR PERFORMING AN ANASTOMOSIS BETWEEN TWO HOLLOW DUCTS IN A PATIENT, IN PARTICULAR BETWEEN THE BLADDER AND THE URETHRA

(30) Priorité: 09.09.2010 FR 1057157
(43) Date de publication de la demande: 17.07.2013
(73) Titulaire: Ferlin, Arnold, 77000 Vaux le Penil (FR)
(72) Inventeur: Ferlin, Arnold, 77000 Vaux le Penil (FR)
(74) Mandataire: Grand, Guillaume
(86) Numéro de dépôt international: PCT/FR2011/052071
(87) Numéro de publication internationale: WO 2012/032279

(56) Documents cités:
- US-A1- 2004 193 185
- US-A1- 2005 010 241
- US-A1- 2005 222 492
- US-A1- 2008 114 385
- US-A1- 2009 281 560

## Description

La présente invention concerne un système de traitement chirurgical pour réaliser une anastomose entre deux conduits creux d'un patient, tels que la vessie et l'urètre du patient.

L'invention a donc trait au domaine de la chirurgie, notamment urologique, viscérale, vasculaire, etc. Elle est par exemple mise en oeuvre après la prostatectomie d'un patient, afin de rétablir chirurgicalement la communication entre le col de la vessie et l'orifice terminal de l'urètre, opposé au méat urétral.

Dans ce dernier contexte, traditionnellement, les chirurgiens réalisent l'anastomose entre la vessie et l'urètre d'un patient à l'aide d'un ou de plusieurs fils de suture, qui sont mis en place à l'aide d'une aiguille. Cette méthode de traitement offre de bons résultats car elle permet de rendre jointive la berge du col de la vessie et la berge de l'orifice terminal de l'urètre, sans avoir à replier ces berges sur elles-mêmes: on peut rétablir une interface de contact direct entre les muqueuses respectives des berges, ce qui est favorable à la cicatrisation et au rétablissement complet et fiable de la communication entre la vessie et l'urètre. Toutefois, la mise en oeuvre de cette méthode demande, à la fois, un temps d'intervention particulièrement important et une grande dextérité pour les chirurgiens, d'autant que la zone d'intervention, à l'arrière du pubis, est particulièrement délicate d'accès et le sphincter doit rester intact tout au long de l'intervention, en évitant notamment sa traction, sa perforation, son écrasement, etc.

Une autre technique connue consiste à replier chacune des berges sur elle-même de façon à, en quelque sorte, former une bride périphérique interne : après avoir accolé ces deux brides, on peut les suturer l'une à l'autre par une série circulaire d'agrafes, mises en place à l'aide d'une sonde urétrale dédiée. EP-A-0 282 157 et US-A-2009/0281560 en fournissent des exemples. Toutefois, les résultats de cette technique sont mitigés : chacune des berges étant repliée sur elle-même, leur muqueuse respective ne sont pas en contact et ne peuvent pas cicatriser en se « soudant » naturellement l'une à l'autre. On comprend la nécessité de prévoir un grand nombre d'agrafes couvrant l'essentiel, voire la totalité de la périphérie de la zone d'anastomose, ces agrafes devant de surcroît être métalliques, ou plus généralement non résorbables, pour garantir la pérennité de l'anastomose. Cependant, la présence prolongée de telles agrafes métalliques engendre souvent des complications dans le domaine urologique, typiquement des calculs.

Ceci étant dit, US-A-2008/114385, qui peut être considéré comme l'art antérieur le plus proche de l'invention revendiquée ici, propose une autre approche et divulgue un système de traitement comprenant une pluralité d'organes de suture conçus pour être rapportés et se fixer par l'intérieur des organes creux à traiter. Chacun de ces organes de suture est constitué de deux branches mobiles, qui sont passées d'une configuration escamotée, dans laquelle les extrémités libres des deux branches sont disposées à une distance radiale de l'axe central d'un corps de support, qui est inférieure au rayon extérieur de ce corps de support, à une configuration déployée, dans laquelle les extrémités libres des branches sont entraînées à l'extérieur du corps, en étant alors situées à une distance radiale de l'axe précité, qui est supérieure au rayon extérieur du corps de support, afin que ces extrémités libres se fixent à la face interne des parois des organes creux à traiter. Cependant, dans la configuration déployée précitée, les extrémités libres des branches des organes de suture enserrent les berges des deux conduits creux jointifs, eh pliant ces berges sur elles-mêmes, empêchant ainsi leur muqueuse respective d'être en contact l'une avec l'autre. Pour les mêmes raisons que développées plus haut, la mise en oeuvre de ce système procure donc des résultats mitigés.

Le but de la présente invention est de proposer un système pour réaliser une anastomose entre deux conduits creux, qui soient fiables et sécurisés, ainsi que faciles et rapides à mettre en oeuvre, tout en obtenant une bonne fixation des conduits.

A cet effet, l'invention a pour objet un système de traitement chirurgical pour réaliser une anastomose, tel que défini à la revendication 1.

Une des idées à la base de l'invention est de chercher à utiliser des organes de suture à la fois bio-résorbables et conformés pour être implantés suivant la direction longitudinale de l'urètre, chacun de ces organes de suture chevauchant la zone d'anastomose entre deux conduits creux d'un patient, tels que l'urètre et le col de la vessie. De cette façon, les berges respectives des deux conduits, typiquement la berge du col de la vessie et la berge de l'orifice terminal de l'urètre, peuvent être rendues jointives, dans le prolongement tubulaire l'une de l'autre, en formant une interface de contact direct entre leur muqueuse respective. Selon l'invention, chacun de ces organes de suture présente à cet effet deux branches spécifiques : l'extrémité libre d'une de ces branches est prévue pour être passée, de l'intérieur vers l'extérieur, au travers de la paroi d'un des conduits creux, tandis que l'extrémité libre de l'autre branche est prévue pour être passée de l'intérieur vers l'extérieur, au travers de la paroi de l'autre conduit creux, puis ces deux extrémités libres sont prévues pour être liées fixement l'une à l'autre par complémentarité de formes soit l'une avec l'autre, soit avec une pièce rapportée de l'organe de suture, autrement dit pour se clipser soit l'une à l'autre, soit toutes les deux à la pièce rapportée précitée. En pratique, à l'opposé de leur extrémité libre précitée, les deux branches sont liées par un pont, qui peut être prévu souple et monobloc avec les branches ou bien qui peut autoriser une mobilité guidée de l'une et/ou de l'autre des branches. Pour mettre en place ces organes de suture, l'invention prévoit d'utiliser un corps de support, que l'on peut ainsi qualifier de « porte-clips » : ce corps de support est conformé pour être placé et immobilisé, durant le temps de déploiement des organes de suture, à l'intérieur des conduits creux, puis pour être dégagé. De plus, pour déformer de façon adéquate les organes de suture en vue de lier fixement l'une à l'autre les extrémités libres de leurs branches, l'invention prévoit qu'un mécanisme agissant sur chacun des organes de suture est porté intérieurement par le corps de support : ce mécanisme peut ainsi être avantageusement commandé par l'intérieur des conduits, notamment à l'aide d'une sonde introduite depuis un orifice débouchant d'un de ces conduits, cette sonde pouvant aussi bien être à commande manuelle, qu'être au moins en partie robotisée.

Des caractéristiques additionnelles avantageuses du système de traitement conforme à l'invention, prises isolément ou selon toutes les combinaisons techniquement possibles, sont spécifiées aux revendications dépendantes 2 à 18.

L'invention sera mieux comprise à la lecture de la description qui va suivre, donnée uniquement à titre d'exemple et faite en se référant aux dessins sur lesquels :
- la figure 1 est une vue en perspective d'un premier mode de réalisation d'un système de traitement conforme à l'invention ;
- la figure 2 est une vue en élévation d'une partie du système de la figure 1 ;
- les figures 3 à 5 sont des coupes respectivement selon les lignes III-III, IV-IV et V-V de la figure 2 ;
- les figures 6 et 7 sont des vues schématiques en élévation, illustrant respectivement deux étapes successives d'une utilisation du système de la figure 1, associée à une vessie d'un patient ;
- la figure 8 est une vue schématique en élévation et partiellement en coupe du système et de la vessie, dans l'étape montrée à la figure 7 ;
- la figure 9 est une vue schématique en élévation du système et de la vessie, illustrant une étape subséquente de l'utilisation de ce système, associée à un urètre du patient, à anastomoser à la vessie ;
- la figure 10 est une vue schématique en perspective, avec coupe partielle, du système, de la vessie et de l'urètre, illustrant une étape subséquente à celle montrée à la figure 9 ;
- la figure 11 est une vue schématique en élévation, avec coupe partielle, du système, de la vessie et de l'urètre, illustrant la même étape que celle montrée à la figure 10;
- les figures 12 à 15 sont des vues similaires à la figure 11, illustrant respectivement des étapes successives de l'utilisation du système ;
- la figure 16 est une vue en élévation d'une partie d'un second mode de réalisation d'un système de traitement conforme à l'invention ;
- la figure 17 est une coupe selon la ligne XVII-XVII de la figure 16 ;
- la figure 18 est une vue en perspective de seulement certains composants montrés sur les figures 16 et 17, dans une configuration différente ;
- les figures 19 à 23 sont des vues schématiques en élévation, illustrant respectivement cinq étapes successives d'une utilisation du système associé à la figure 16, mises en oeuvre sur la vessie d'un patient, représentée schématiquement en coupe longitudinale ; et
- les figures 24 à 28 sont des vues schématiques en élévation, illustrant respectivement cinq étapes successives, subséquentes à l'étape montrée à la figure 23, avec coupe longitudinale de la vessie et d'un urètre à anastomoser à la vessie.

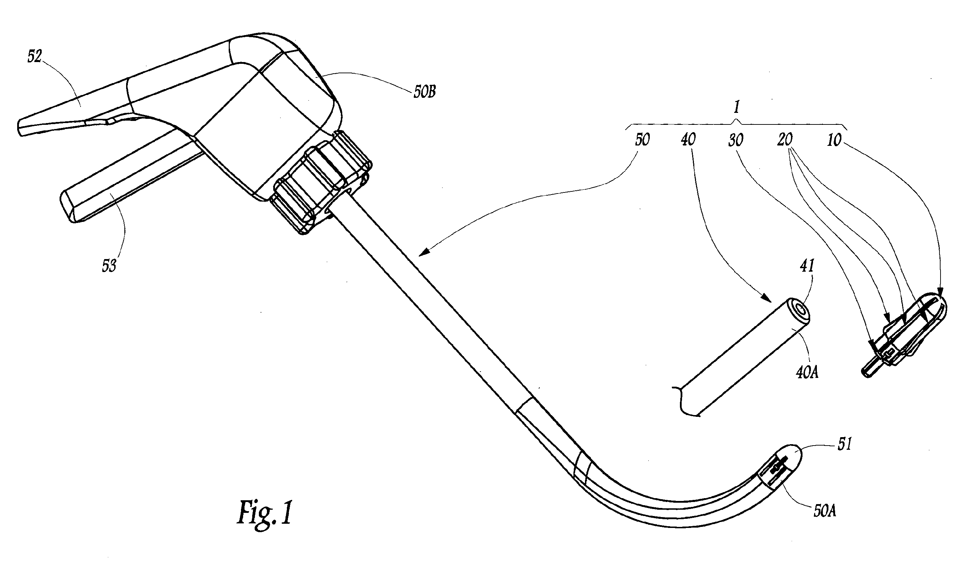

Sur la figure 1 est représenté un système 1 de traitement chirurgical pour réaliser une anastomose entre la vessie et l'urètre d'un patient. Ce système 1 comprend principalement :
- un corps 10 qui, comme détaillé ci-après, supporte un ou une pluralité d'éléments de suture 20 et dans lequel est agencé un mécanisme 30 de sollicitation de ces éléments de suture,
- un applicateur 40 conçu pour mettre en place le corps 10 lors de l'utilisation du système 1, et
- une sonde urétrale 50 destinée, lors de l'utilisation du système 1, à commander le mécanisme 30.

L'applicateur 40 et la sonde 50 seront décrits plus loin, lors de la présentation détaillée d'un exemple d'utilisation du système 1.

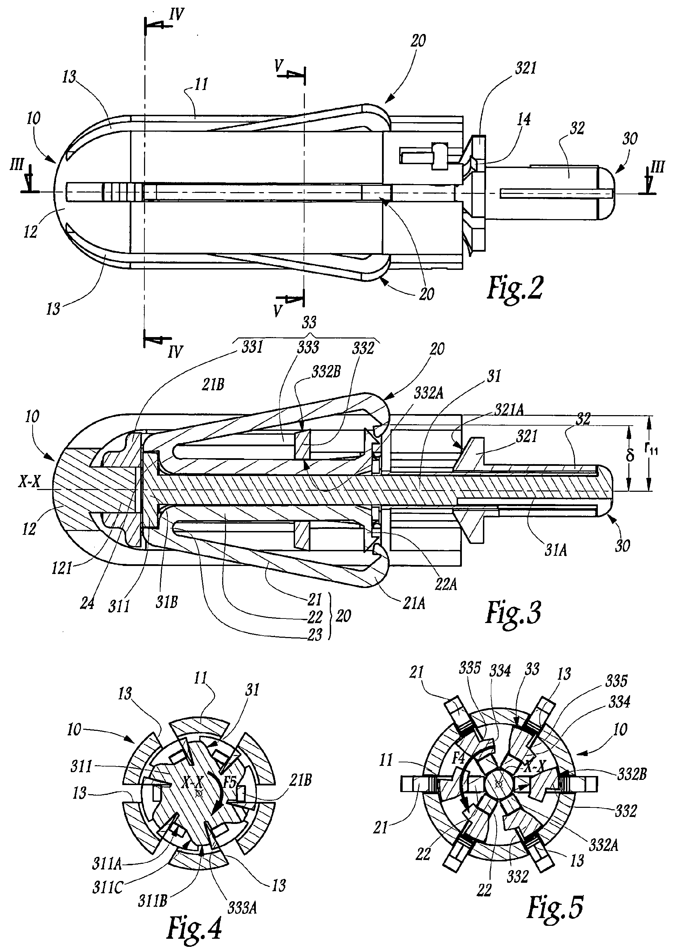

Comme visible sur la figure 1 et comme représenté plus en détail sur les figures 2 à 5, le corps 10 présente une forme globalement tubulaire, centrée sur un axe longitudinal X-X. Dans l'exemple de réalisation considéré ici, le corps 10 comporte une jupe cylindrique 11 à base circulaire centrée sur l'axe X-X. A son extrémité distale, cette jupe 11 est fermée par une paroi 12 en forme d'ogive. A son extrémité proximale, la jupe 11 est axialement ouverte sur l'extérieur. De plus, le volume interne de la jupe 11 communique avec l'extérieur par plusieurs fentes traversantes 13, qui s'étendent en longueur suivant la direction de l'axe X-X et qui sont réparties de manière sensiblement régulière suivant la périphérie de la jupe. Dans l'exemple de réalisation considéré sur les figures, ces fentes 13 sont au nombre de six, comme bien visible sur les figures 4 et 5. Leur intérêt apparaîtra plus loin.

Comme bien visible sur la figure 3, chacun des éléments de suture 20 présente la forme globale d'un U. Plus précisément, chaque élément 20 inclut deux branches allongées 21 et 22 qui, à leur extrémité distale, sont liées à demeure l'une à l'autre par un pont transversal 23. Ce pont 23 relie ainsi les branches 21 et 22 de manière élastiquement déformable, dans le sens où, à la façon d'une liaison souple à charnière globalement centrée sur le pont 23, les branches 21 et 22 peuvent être écartées ou rapprochées l'une vis-à-vis de l'autre, moyennant la déformation élastique de la matière constituant l'élément 20, sans rupture de ce dernier, qui forme ainsi une pièce d'un seul tenant.

A l'opposé du pont 23, les branches 21 et 22 présentent respectivement des extrémités proximales libres 21A et 22A. Comme bien visible sur la figure 3, ces extrémités libres 21 A et 22A sont conformées de manière sensiblement complémentaire afin de pouvoir être mises en prise mécanique l'une avec l'autre et ainsi fixer les branches 21 et 22 l'une à l'autre au niveau de leur extrémité proximale. Dans l'exemple de réalisation considéré sur les figures, l'extrémité libre 22A de la branche 22 est conformée en un cran anguleux, qui s'étend en saillie depuis le reste de la branche 22 en direction de la branche 21, tandis que l'extrémité 21A de la branche 21 est conformée en une encoche, qui est délimitée en creux dans la face de l'extrémité 21A, tournée vers la branche 22, et qui est dimensionnée pour recevoir de manière ajustée le cran d'extrémité de la branche 22. Plus généralement, on comprend que les extrémités libres 21A et 22A des branches 21 et 22 présentent des motifs en reliefs et/ou en creux complémentaires, à même de coopérer pour fixer mécaniquement les branches 21 et 22 l'une à l'autre lorsque ces extrémités 21 A et 22A sont suffisamment appuyées l'une contre l'autre, en particulier suivant une direction transversale à la direction longitudinale des branches 21 et 22.

En pratique, les éléments de suture 20 sont réalisés en un matériau qui autorise leur déformation souple, comme évoqué plus haut. De surcroît, ce matériau est choisi comme étant bio-résorbable, c'est-à-dire un matériau qui peut se résorber sous l'action physico-chimique de tissus vivants en contact desquels ce matériau est placé. A titre d'exemple non limitatif, le matériau constituant les éléments de suture 20 est un polymère biorésorbable, tel que l'acide polylactique (PLA, PLLA), l'acide polyglycolique (PGA) ou le polydioxanone (PDO).

Avantageusement, pour des raisons qui apparaîtront plus loin, la branche 21 présente, à son extrémité distale, un prolongement 21 B qui forme avec le côté distal du pont 23 un épaulement 24.

Dans la configuration des éléments de suture 20 montrée aux figures 1 à 5, les différents éléments de suture 20, qui sont ici au nombre de six, sont agencés pour l'essentiel à l'intérieur du corps 10, avec leurs branches 21 et 22 qui s'étendent en longueur globalement suivant la direction de l'axe X-X. Plus précisément, comme bien visible sur les figures 3 et 5, la branche 22 et le pont 23 de chaque élément 20 sont disposés en totalité à l'intérieur du volume libre de la jupe 11, tandis que la branche 21 est reçue, avec une légère inclinaison, dans l'une des fentes 13, en émergeant quelque peu à l'extérieur de la jupe 11, dans sa partie proximale. Lors de l'utilisation du système 1, chaque élément de suture 20 est à même d'être dégagé en totalité du corps 10, en passant par l'une des fentes 13, globalement suivant une direction radiale à l'axe X-X. Avantageusement, à des fins de guidage des éléments 20 lors de ce dégagement, la largeur des fentes 13, c'est-à-dire l'écartement entre les bords longitudinaux de chacune de ces fentes, est sensiblement égale à l'épaisseur des branches 21 et 22 : autrement dit, en coupe transversale à l'axe X-X, les branches 21 et 22 de chaque élément 20 présentent une dimension orthoradiale à l'axe X-X sensiblement égale à l'écartement orthoradiale des fentes 13, comme bien visible sur la figure 5.

Le mécanisme 30 comporte trois pièces principales, qui sont toutes centrées sur l'axe X-X, en étant agencées à l'intérieur du corps 10 ou, à tout le moins, dans le prolongement axial du volume interne de ce corps du côté proximal de ce dernier. Ces trois pièces sont :
- une tirette 31 qui, dans l'exemple considéré sur les figures, est constituée d'une tige rectiligne centrée sur l'axe X-X, en s'étendant sensiblement sur toute la longueur de la jupe 11 et en se prolongeant, par son extrémité proximale 31A, à l'extérieur du corps 10,
- un poussoir 32 qui, dans l'exemple considéré, est réalisé sous la forme d'un tube agencé co-axialement autour de l'extrémité proximale 31A de la tirette 31, et
- un guide 33 qui, dans l'exemple considéré, inclut, d'une part, à son extrémité distale, un embout tubulaire 331 qui est agencé co-axialement autour d'un téton complémentaire 121 s'étendant en saillie depuis la paroi 12 du corps 10 vers l'intérieur de ce corps formant tous les deux une liaison pivot, et, d'autre part, six portions de disque 332, qui, comme bien visible sur la figure 5, sont réparties de manière sensiblement régulière autour de l'axe X-X, en étant toutes situées dans un même plan perpendiculaire à cet axe, passant par la région médiane de la jupe 11, étant précisé que chacune de ces portions 332 est reliée rigidement à l'embout distal 331 par une barrette 333 qui s'étend parallèlement à l'axe X-X, le long de la face intérieure de la jupe 11.

A son extrémité distale 31 B, la tirette 31 est pourvue d'une tête saillante annulaire 311 centrée sur l'axe X-X. Comme bien visible sur la figure 4, cette tête 311 présente, en coupe transversale à l'axe X-X, un profil extérieur élaboré, dans le sens où ce profil n'est pas rigoureusement circulaire : ainsi, ce profil présente six motifs qui se répètent à l'identique autour de l'axe X-X les uns à la suite des autres, chaque motif incluant, d'une part, un premier segment 311A s'étendant suivant une direction orthoradiale à l'axe X-X et, d'autre part, un second segment 311 B, s'étendant lui-aussi de manière orthoradiale à l'axe X-X mais en étant radialement plus éloigné de l'axe X-X que le segment 311A, étant précisé que les segments 311A et 311 B sont reliés l'un à l'autre par un chemin de came 311C.

La tirette 31 est montée dans le corps 10 à la fois à translation selon l'axe X-X et à rotation sur elle-même autour de cet axe X-X. En pratique, un palier d'accouplement ou un organe de guidage similaire est avantageusement interposé radialement entre la tirette 31 et la jupe 11 du corps 10.

Le poussoir 32 est, quant à lui, monté sur l'extrémité proximale 31A de la tirette 31 à translation selon l'axe X-X.

Par ailleurs, le guide 33 est monté dans le corps 10 à rotation sur lui-même autour de l'axe X-X. Dans l'exemple de réalisation considéré sur les figures, le guide 33 est ainsi guidé en rotation par la coopération entre son embout distal 331 et le téton 121 de la paroi distale 12 du corps 10.

Avantageusement, le guide 33 et la tirette 31 sont liés en rotation l'un à l'autre, pour des raisons qui apparaîtront plus loin : dans l'exemple de réalisation, cette liaison cinématique rotative entre le guide 33 et la tirette 31 est réalisée par la coopération mécanique entre la tête 311 de la tirette et les extrémités distales 333A des barrettes 333, comme bien visible sur la figure 4.

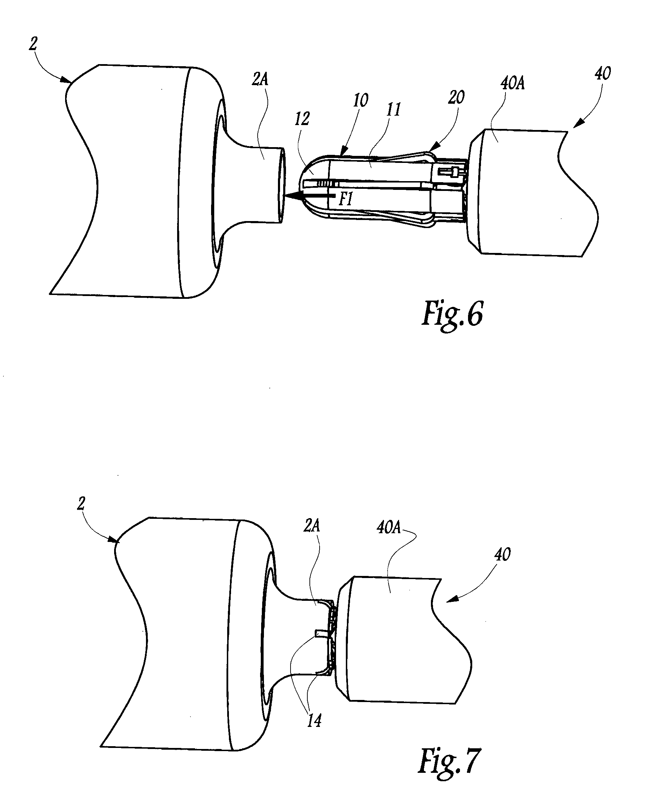

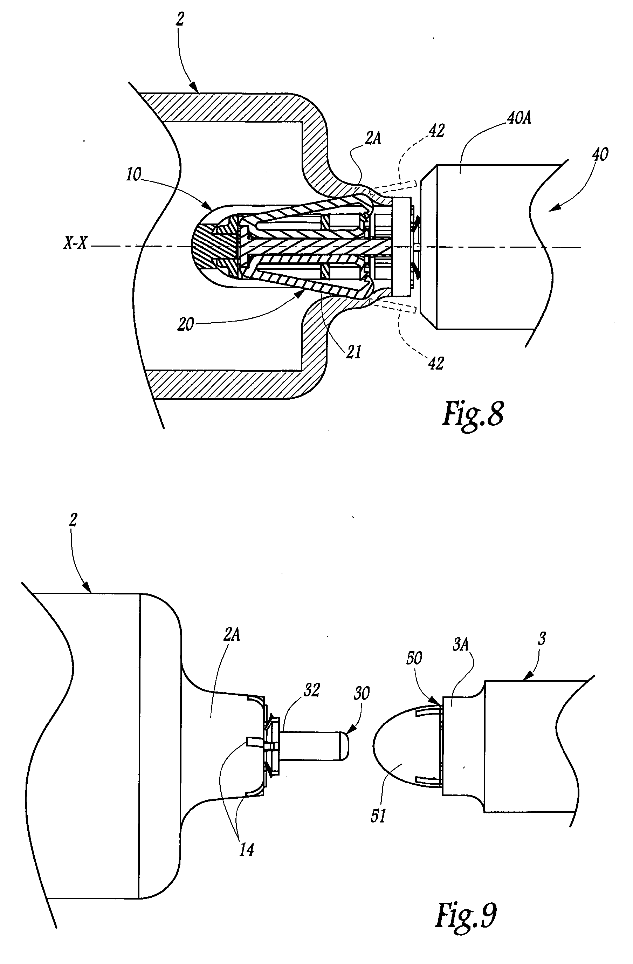

On va maintenant décrire en détail un exemple d'utilisation du système 1, en vue de réaliser une anastomose entre la vessie 2 d'un patient, visible sur les figures 6 à 15, et l'urètre 3 de ce patient, visible sur les figures 9 à 15. Typiquement, le patient précité est traité à l'aide du système 1 après avoir subi une prostatectomie, typiquement une vésiculoprostatectomie totale, ou prostatectomie radicale.

Initialement, on considère que le corps 10, les éléments de suture 20 et le mécanisme 30 sont mis à la disposition du chirurgien dans leur configuration relative représentée sur les figures 1 à 5. Plus précisément, comme bien visible sur la figure 3, chacun des six éléments de suture 20 est agencé dans le corps 10 de sorte que sa branche 22 court le long de la tirette 31, en se trouvant radialement interposée entre cette tirette et l'une des portions 332 du guide 33. Plus précisément, chacune des portions 332 du guide 33 délimite, sur sa face tournée vers l'axe X-X, une surface 332A d'immobilisation radiale de la branche 22, comme bien visible sur la figure 5. Dans le même temps, la branche 21 de chaque élément de suture 20 s'étend en regard de la branche 22, avec interposition entre ces branches d'une des portions 332. De plus, dans cette configuration initiale, les extrémités libres 21 A et 22A des branches 21 et 22 sont distantes l'une de l'autre, c'est-à-dire que, plus généralement, ces deux extrémités libres ne coopèrent pas l'une avec l'autre. Egalement dans cette configuration initiale, la tête 311 de la tirette 31 se trouve logée dans les épaulements respectifs 24 des éléments de suture 20, avec le prolongement distal 21 B de chaque branche 21 sensiblement en appui radial sur l'un des segments 311 A du profil transversal de cette tête 311, comme bien visible sur la figure 4.

Dans un premier temps, le corps 10, muni des éléments de suture 20 et du mécanisme 30 agencés selon la configuration initiale détaillée ci-dessus, est manipulé à l'aide de l'applicateur 40, comme représenté sur la figure 6. Pour ce faire, l'extrémité proximale 31 A de la tirette 31 est engagée et retenue dans un logement complémentaire 41 (figure 1) délimité à l'extrémité distale 40A de cet applicateur 40. Puis, l'applicateur 40 est manipulé par le chirurgien, typiquement dans le cadre d'une chirurgie endoscopique ou d'une chirurgie ouverte, pour rapprocher le corps 10 de la vessie 2, jusqu'à introduire le corps 10 à l'intérieur du col 2A de cette vessie 2, en centrant l'axe X-X à l'intérieur de ce col 2A et en y engageant d'abord la paroi distale 12, comme indiqué par la flèche F1 sur la figure 6. La forme en ogive de la paroi 12 facilite cette mise en place.

Préalablement à cette mise en place du corps 10, le col 2A de la vessie peut être à reconstruire chirurgicalement. En effet, au niveau des berges de la vessie, le col peut ne pas être prêt, dans le sens où il est trop large ou non circulaire par exemple. Dans ce cas, le chirurgien reforme le col 2A par un geste connu en soi, soit à l'aide d'un fil et d'une aiguille, soit en utilisant l'applicateur 40 pourvu de moyens ad hoc, non représentés sur les figures du premier mode de réalisation.

En pratique, diverses formes de réalisation, notamment mécaniques et/ou magnétiques, sont envisageables pour connecter mécaniquement l'extrémité distale 40A de l'applicateur 40 à l'extrémité proximale 31A de la tirette 31, voire, plus généralement, à une extrémité proximale du mécanisme 30. Dans tous les cas, les moyens d'accouplement correspondants sont amovibles, dans le sens où, via une commande ad hoc transmise par l'applicateur 40 depuis son extrémité proximale, le mécanisme 30 peut être déconnecté vis-à-vis de l'applicateur 40, permettant ainsi de dégager cet applicateur tout en laissant en place le corps 10 à l'intérieur du col 2A de la vessie 2.

Avantageusement, le corps 10 est pourvu de moyens mécaniques permettant d'immobiliser de manière amovible le corps 10 vis-à-vis du col 2A de la vessie 2. A titre d'exemple, comme représenté de manière schématique sur la figure 7, de tels moyens d'immobilisation incluent des crochets mobiles 14 : dans la configuration initiale montrée sur les figures 1 à 5, ces crochets 14 sont escamotés à l'intérieur du volume libre du corps 10, en particulier au niveau de l'extrémité proximale de ce corps, puis, après positionnement du corps 10 dans le col 2A de la vessie 2 à l'aide de l'applicateur 40, ces crochets 14 sont escamotés de manière à venir se fixer, notamment par agrippement ou par pincement, à la paroi constituant le col 2A de la vessie 2. Avantageusement, la commande du déploiement des crochets 14 est réalisée à l'aide de l'applicateur 40.

Bien entendu, d'autres formes de réalisation que les crochets 14 sont envisageables pour ce qui concerne la fixation temporaire du corps 10 au col 2A de la vessie 2. En particulier, suivant une variante non représentée, moins élaborée que les crochets mobiles 14, le chirurgien peut utiliser un dispositif de fixation rapporté, tel qu'un fil chirurgical par exemple.

Suivant une option particulièrement avantageuse, l'applicateur 40 est pourvu, à son extrémité distale 40A, d'éléments de coupe 42, visibles uniquement en pointillés sur la figure 8. Ces éléments de coupe 42, typiquement des petites lames de coupe ou des pointes biseautées, sont répartis suivant la périphérie de l'extrémité distale 40A de l'applicateur 40, en étant avantageusement prévus pour être déployés en saillie depuis cette extrémité distale 42A en direction du corps 10. On notera que le positionnement angulaire de ces éléments de coupe 42 est lié au positionnement angulaire, autour de l'axe X-X, des éléments de suture 20 : lorsque le corps 10 est connecté à l'applicateur 40, les éléments de coupe 42 sont respectivement situés axialement en regard d'un des éléments de suture 20, autrement dit d'une des fentes 13. De la sorte, après avoir mis en place le corps 10 dans le col 2A de la vessie 2 à l'aide de l'applicateur 40 et avant de dégager cet applicateur, les éléments de coupe 42 sont utilisés, notamment en étant déployés sous l'action d'une commande ad hoc transmise par l'applicateur 40 depuis son extrémité proximale, pour découper localement la paroi constituant le col 2A de la vessie 2, suivant une direction transversale à ce col et depuis l'extérieur de ce col. De la sorte, les six portions de la paroi du col 2A de la vessie 2, qui sont respectivement situées radialement en regard des fentes 13 et intérieurement contre lesquelles s'appuient légèrement les branches respectives 21 des éléments de suture 20, sont extérieurement affaiblies sous l'action des éléments de coupe 42. Le cas échéant, l'action des éléments de coupe 42 est telle que les portions précitées de la paroi constituant le col 2A de la vessie 2 se trouvent ajourées sur sensiblement toute leur épaisseur, ce qui revient à dire que les éléments de coupe 42 arrivent alors sensiblement au contact des branches 21 des éléments de suture 20, sans toutefois endommager ces branches.

Dans un second temps opératoire, après dégagement de l'applicateur 40, le chirurgien utilise la sonde urétrale 50, comme représenté sur la figure 9. En pratique, cette sonde est introduite dans l'urètre 3 depuis le méat de ce dernier, jusqu'à un orifice terminal opposé 3A de l'urètre, visible sur la figure 9. On comprend que cet orifice terminal 3A résulte d'une résection antérieure de l'urètre en sa zone membranaire, réalisée notamment dans le cadre d'une prostatectomie.

Comme bien visible sur la figure 9, la sonde urétrale 50 est mise en place dans l'urètre 3, de manière que son extrémité distale 50A soit située au niveau de l'orifice terminal 3A de l'urètre 3. Pour faciliter cette mise en place, l'extrémité distale 50A de la sonde 50 est avantageusement pourvue d'un capuchon bombé 51 qui, une fois la sonde 50 positionnée, est dégagé par le chirurgien par un outil additionnel ad hoc.

A titre d'option non représentée, avant ou après dégagement du capuchon 51, l'extrémité distale 50A de la sonde 50 peut être immobilisée de manière amovible dans l'orifice terminal 3A de l'urètre 3, notamment par des moyens fonctionnellement, voire structurellement similaires aux crochets 14 décrits plus haut pour immobiliser temporairement le corps 10 dans le col 2A de la vessie 2. Le cas échéant, le capuchon 51 est intérieurement pourvu de moyens facilitant ou participant à cette fixation amovible.

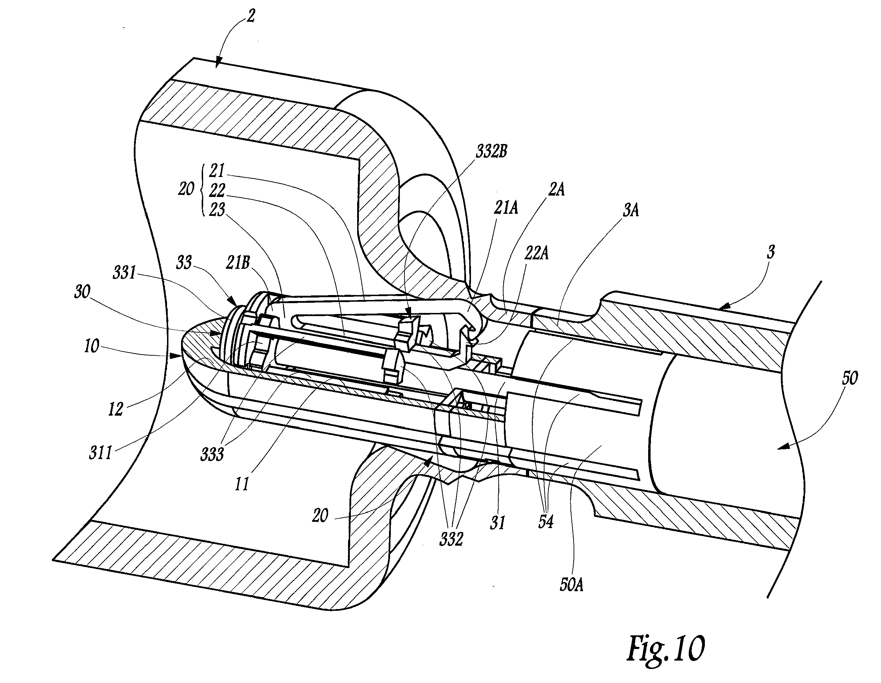

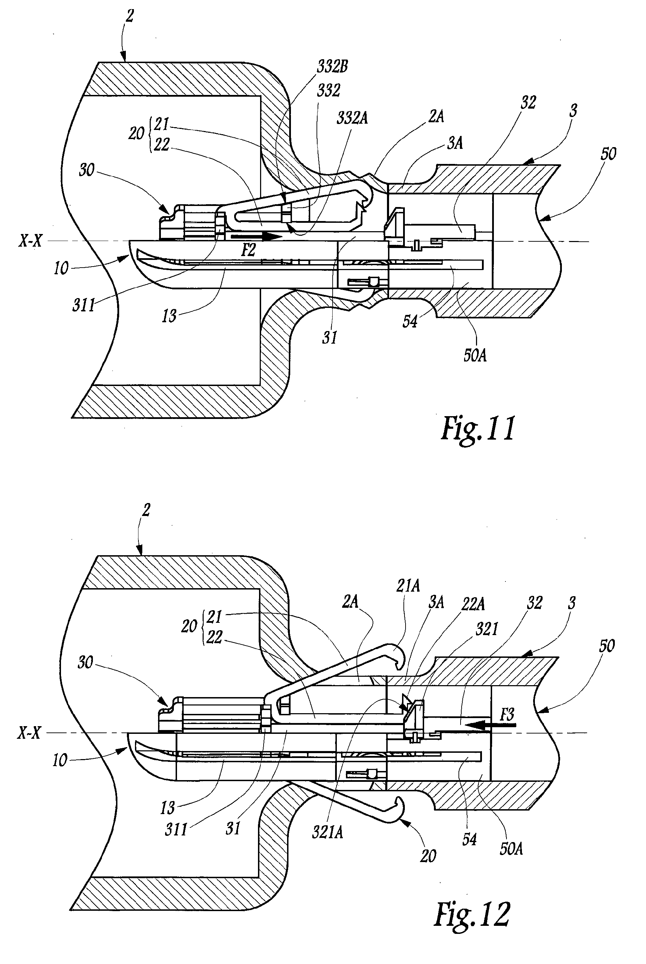

Lors d'un temps chirurgical subséquent, illustré aux figures 10 et 11, le chirurgien rapproche l'un de l'autre le col 2A de la vessie 2 et l'orifice 3A de l'urètre 3. En pratique, notamment afin de ne pas endommager le sphincter de l'urètre 3, c'est préférentiellement la vessie 2 qui est sollicitée par le chirurgien pour rapprocher son col 2A de l'orifice 3A de l'urètre 3. Dans tous les cas, la berge du col de la vessie et la berge de l'orifice de l'urètre se retrouvent accolées de manière jointive, dans le prolongement tubulaire l'une de l'autre, comme représenté sur les figures 10 et 11. Ce faisant, l'extrémité proximale 31A de la tirette 31 se trouve engagée mécaniquement à l'intérieur de l'extrémité distale 50A de la sonde 50 : de manière non représentée en détail sur les figures, cette extrémité distale 50A est en effet conformée pour être assemblée de manière complémentaire à l'extrémité proximale 31 A de la tirette 31, à des fins de commande du mécanisme 30. Autrement dit, des aménagements intérieurs de l'extrémité 50A de la sonde 50 sont prévus pour se connecter mécaniquement au mécanisme 30, de manière à autoriser la commande de l'entraînement de ce mécanisme vis-à-vis du corps 10. En pratique, on comprend que diverses formes de réalisation sont envisageables à cet égard du moment que la sonde 50 soit à même de transmettre des mouvements de commande au mécanisme 30 depuis son extrémité proximale 50B, visible sur la figure 1. Les moyens mécaniques de transmission correspondants, intégrés à la sonde, sont bien connus dans le domaine de l'instrumentation chirurgicale et, au besoin, le lecteur pourra se reporter à la littérature technique concernée. D'ailleurs, dans l'exemple de réalisation considéré sur cette figure 1, l'extrémité proximale 50B est réalisée sous la forme d'un mécanisme à actionnement manuel, incluant une poignée fixe 52 et une gâchette mobile 53. A titre de variante non représentée, l'extrémité proximale 50B de la sonde 50 est reliée mécaniquement à une interface connectable à un bras robotisé, permettant une commande du mécanisme 30 pilotée par un ordinateur placé sous la supervision du chirurgien, typiquement dans le cadre d'une chirurgie assistée par ordinateur.

Ainsi, en revenant aux figures 10 et 11, on constate que, jusqu'ici, les éléments de suture 20 et le mécanisme 30 occupent toujours la même configuration initiale, montrée sur les figures 1 à 5. On peut qualifier cette configuration de configuration escamotée pour ce qui concerne les éléments de suture 20 : en effet, comme exposé plus haut, les extrémités libres 21A et 22A des branches 21 et 22 de chaque élément de suture 20 sont disposées à l'intérieur du corps 10 et donc à l'intérieur du col 2A de la vessie 2, sans coopérer l'une avec l'autre. Autrement dit, dans cette configuration, les extrémités 21 A et 22A des branches 21 et 22 sont, au maximum, distantes radialement de l'axe X-X d'une valeur notée δ sur la figure 3, qui est inférieure au rayon extérieur r₁₁ de la jupe 11 du corps 10. On notera que, pour des raisons de visibilité, seuls deux des six éléments 20 sont représentés sur les figures 10 et 11, ainsi que sur les figures 12 à 15 commentées plus bas.

A partir de cette configuration escamotée, le chirurgien commande la déformation des éléments de suture 20 jusqu'à une première configuration intermédiaire, représentée à la figure 12. Pour ce faire, le chirurgien commande, via la sonde urétrale 50, l'entraînement de la tirette 51 en translation selon l'axe X-X, dirigée vers le chirurgien, c'est-à-dire dirigée à l'opposé de la paroi distale 12 du corps 10, comme indiqué par la flèche F2 sur la figure 11. Par appui axial de la tête 311 contre l'épaulement 24 de chaque élément de suture 20, ces éléments de suture sont entraînés suivant un mouvement de translation correspondant, comme bien visible par comparaison des figures 11 et 12, le cas échéant jusqu'à venir appuyer axialement les branches 22 contre le poussoir 32 et entraîner librement ce poussoir en translation autour de la tirette 31, comme représenté sur la figure 12. Dans le même temps, en raison de l'immobilité axiale du guide 33 à l'intérieur du corps 10, le pont transversal 23 de chacun des éléments de suture 20 est rapproché axialement des portions 332 du guide 33 : chacune de ces portions 332 forme alors, en quelque sorte, un coin d'écartement relatif entre les branches 21 et 22. Plus précisément, tandis que chacune de ces portions d'écartement 332 maintient radialement en place la branche 22, par appui radial de sa surface 332A contre cette branche, sa surface radialement opposée 332B forme une rampe pour la branche 21, forçant l'écartement de cette branche vis-à-vis de la branche 22 au fur et à mesure que l'élément de suture est translaté sous l'action de la tirette 31. L'extrémité libre 21 A de la branche 21 est ainsi déplacée transversalement à la paroi constituant le col 2A de la vessie 2 et traverse de part en part cette paroi, de l'intérieur vers l'extérieur. Le passage de chacune des branches 21 à travers la paroi du col de la vessie est facilité par le fait que les six portions de cette paroi ainsi traversées ont été préalablement affaiblies par les éléments de coupe 42, comme expliqué plus haut.

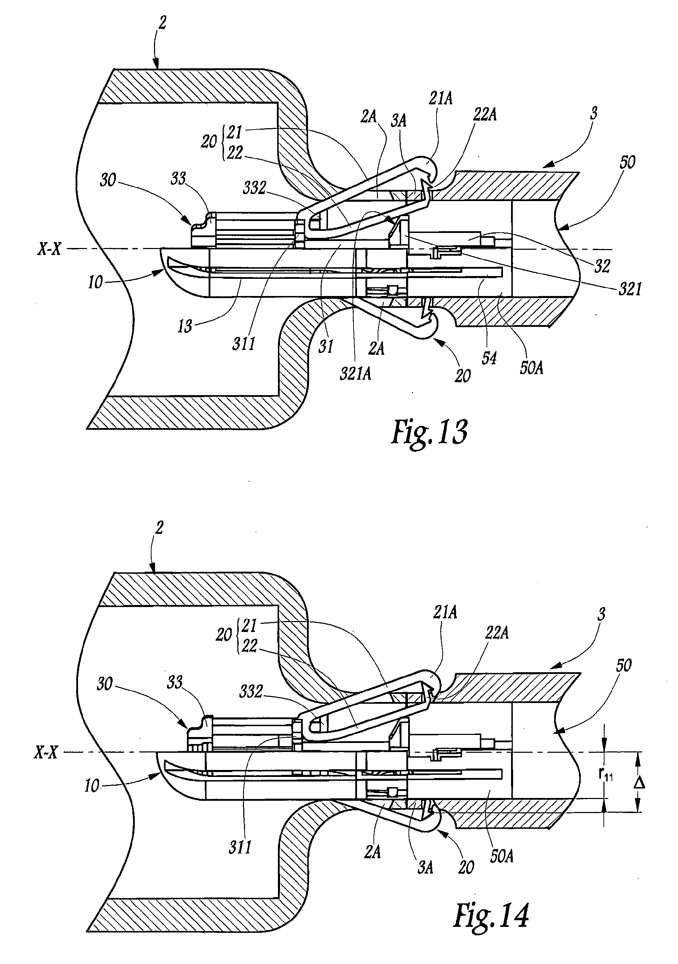

Puis, le chirurgien commande, via la sonde urétrale 50, le déplacement du poussoir 32 en translation selon l'axe X-X, en direction de la paroi 12 du corps 10, comme indiqué par la flèche F3 sur la figure 12. Le poussoir 32 tend alors à entraîner la branche 22 selon un mouvement de translation correspondant. Cependant, eu égard à la résistance de la tête 311 de la tirette 31, alors commandée pour être immobile le long de l'axe X-X, la branche 22 se déforme. Plus précisément, à son extrémité distale, le poussoir 32 est pourvu d'une tête 321 délimitant, sur sa face distale, une surface de rampe 321A pour les branches 22. Dans l'exemple de réalisation considéré ici, cette surface de rampe 321A est sensiblement tronconique, centrée sur l'axe X-X et convergente vers la paroi 12 du corps 10. Par effet de rampe, cette tête 321 du poussoir 32 déforme la branche 22 radialement vers l'extérieur, rapprochant ainsi radialement son extrémité libre 22A de la branche 21, en faisant traverser à cette extrémité libre 22A la paroi de l'orifice terminal 3A de l'urètre 3, de l'intérieur vers l'extérieur, comme représenté sur la figure 13. Les éléments de suture 20 occupent alors une seconde configuration intermédiaire.

On remarquera que la déformation des branches 22, en particulier la trajectoire des extrémités libres 22A, est guidée par des fentes traversantes 54 délimitées dans l'extrémité distale 50A de la sonde 50, ces fentes 54 étant respectivement prévues pour s'étendre dans le prolongement rectiligne d'une des fentes 13 du corps 10, moyennant le positionnement angulaire adéquat entre l'extrémité distale 50A de la sonde 50 et le corps 10 lors de l'assemblage de la sonde 50 au mécanisme 30, comme bien visible sur la figure 10.

On remarquera également que la forme pointue, en direction de la branche associée 21, de l'extrémité libre 22A de chaque branche 22 facilite le passage de cette extrémité libre à travers la paroi délimitant l'orifice 3A de l'urètre 3, permettant en effet la perforation transversale de cette paroi.

Ainsi, dans leur seconde configuration intermédiaire représentée à la figure 13, les éléments de suture 20 ont les extrémités libres 21 A et 22A de leurs branches 21 et 22 disposées toutes les deux à l'extérieur du corps 10, ainsi que de la vessie 2 et de l'urètre 3, sans pour autant que ces extrémités libres coopèrent l'une avec l'autre.

Le chirurgien va alors commander la mise en prise l'une avec l'autre des extrémités libres 21 A et 22A des branches 21 et 22 de chaque élément de suture 20, pour aboutir à ce que ces éléments de suture soient dans une configuration dite déployée, représentée à la figure 14. Pour ce faire, le chirurgien commande, via la sonde urétrale 50, l'entraînement en rotation du guide 33 et de la tirette 31.

Ainsi, pour ce qui concerne le guide 33, la mise en rotation de ce dernier, qui est indiqué par la flèche F4 sur la figure 5, conduit à dégager, suivant une direction orthoradiale à l'axe X-X, chacune des portions 332 d'entre les branches 21 et 22 de chaque élément de suture 20. Autrement dit, via cette mise en rotation du guide 33, la surface de rampe 332B de chaque portion d'écartement 332 s'efface othoradialement vis-à-vis de la branche 21 : par rappel élastique, cette branche 21 tend alors à se rapprocher de la branche 22, en engageant son extrémité libre 21 A avec l'extrémité libre 22A de la branche 22. Avantageusement, pendant ce rappel élastique de la branche 21, la branche 22 est maintenue radialement immobile par la surface 332A de la portion d'écartement 332 : pour ce faire, comme bien visible sur la figure 5, chaque portion d'écartement 332 est pourvue d'un talon 334, qui prolonge, suivant une direction périphérique à l'axe, la surface d'immobilisation 332A et qui, à l'opposé, est relié à la surface de rampe 332B par un épaulement en creux 335 dans laquelle la branche 21 est reçue. L'engagement mécanique de l'extrémité libre 21A avec l'extrémité libre 22A est alors franc et fiable.

Pour ce qui concerne la tirette 31, sa mise en rotation autour de l'axe X-X, qui est indiquée par la flèche F5 sur la figure 4, conduit le prolongement distal 21 B de chaque branche 21 à suivre le chemin de came 311C du profil transversal de la tête 311 de cette tirette. Autrement dit, chaque prolongement distal 21 B est déplacé, par effet de came, depuis le segment 311 A au segment 311 B du profil transversal de la tête 311, en étant ainsi entraîné radialement vers l'extérieur du corps 10. Ce déplacement par effet de came sollicite chaque branche 21 à la façon d'un levier, dans le sens où l'entraînement radialement vers l'extérieur de l'extrémité distale de chaque branche 21 induit un basculement radialement vers l'intérieur de son extrémité libre 21 A, favorisant l'engagement mécanique de cette extrémité libre avec l'extrémité libre 22A de la branche 22. Ainsi, les extrémités 21A et 22A des branches 21 et 22 se retrouvent distantes radialement de l'axe X-X d'une valeur notée Δ sur la figure 14, qui est supérieure au rayon extérieur r₁₁ de la jupe 11 du corps 10.

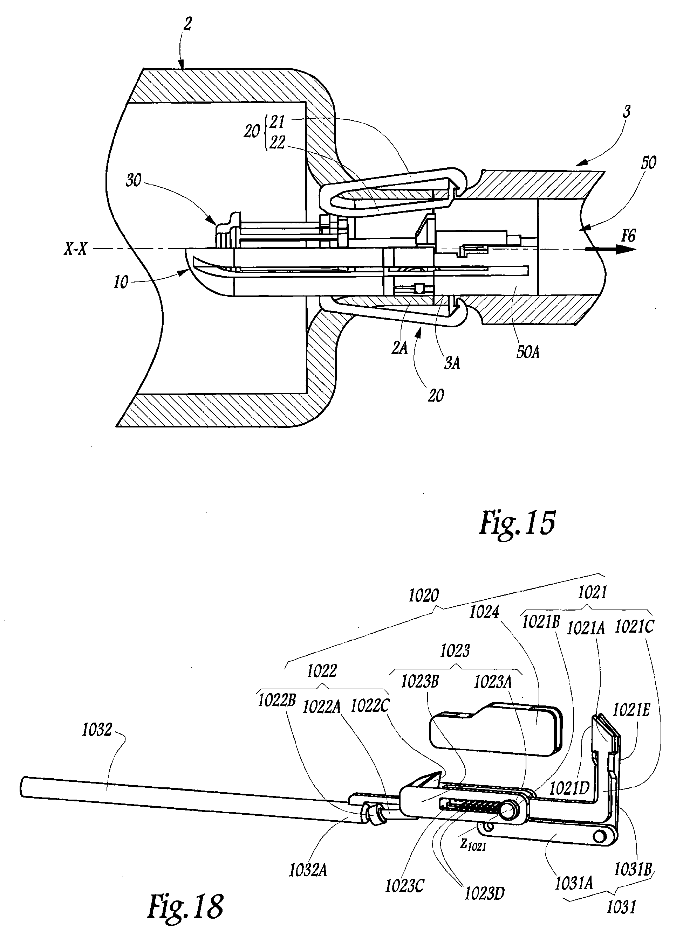

Il reste alors au chirurgien à finir de dégager en totalité les éléments de suture 20 vis-à-vis du corps 10. Pour ce faire, il commande, via la sonde urétrale 50, la poursuite de la rotation de la tirette 31 et du guide 33 : au niveau axial de la tête 311, les extrémités distales 333A des barrettes 33 poursuivent le dégagement radial vers l'extérieur de l'épaulement 24 de chaque élément de suture 20, tandis que, au niveau axial des portions d'écartement 332, les surfaces d'immobilisation 332A s'effacent vis-à-vis des branches 22 qui, par rappel élastique vis-à-vis du reste des éléments de suture 20, tendent à se déformer radialement vers l'extérieur. Le système 1 se trouve alors dans l'état représenté sur la figure 15, avec les éléments de suture 20 dans une configuration libérée vis-à-vis du corps 10 et du mécanisme 30. Le corps 10 peut alors être dégagé, en étant évacué par l'intérieur de l'urètre 3, par entraînement avec la sonde urétrale 50, comme indiqué par la flèche F6 sur la figure 15.

L'intervention chirurgicale prend alors fin. Les éléments de suture 20 restent en place dans leur configuration déployée, en chevauchant chacun l'interface d'anastomose réalisée entre le col 2A de la vessie 2 et l'orifice terminal 3A de l'urètre 3. On remarquera que, avantageusement, la face des extrémités libres 21 A des branches 21, opposée à la branche 22 associée, est prévue arrondie, en particulier non coupante et non perforante, pour ne pas a posteriori endommager les tissus biologiques environnants.

Par la suite, les éléments de suture 20 vont progressivement être résorbés, après cicatrisation de l'anastomose, dont la qualité est remarquable eu égard au contact direct entre la muqueuse du col de la vessie et la muqueuse de l'orifice terminal de l'urètre.

Divers aménagement et variantes au système 1 et à la méthode de mise en oeuvre de ce système sont par ailleurs envisageables. A titre d'exemples :
- Plutôt que de déformer d'abord la branche 21 puis la branche 22 de chaque élément de suture 20, le mécanisme 30 peut être aménagé pour déformer d'abord la branche 22 puis la branche 21 ;
- Plutôt que d'être commandés en rotation conjointement, la tirette 31 et le guide 33 peuvent être commandés en rotation de manière indépendante, notamment selon les souhaits du chirurgien ;
- A titre optionnel, le système 1 inclut des moyens pour affaiblir localement la paroi de l'orifice terminal 3A de l'urètre 3 afin de faciliter ensuite le passage traversant pour les extrémités libres 22A des branches 22 ; en pratique, de tels moyens d'affaiblissement sont avantageusement intégrés dans l'extrémité distale 50A de la sonde et/ou dans le capuchon 51 ; en présence de tels moyens d'affaiblissement, la forme pointue de ces extrémités libres 22A peut alors être moins marquée, voire omise ;

- A titre optionnel, l'extrémité proximale du corps 10 et l'extrémité distale de la sonde 50 peuvent être conçues pour coopérer directement l'une avec l'autre par complémentarité de formes ; de cette façon, la liaison mécanique entre le corps 10 et la sonde 50 s'en trouve renforcée et/ou davantage contrainte en positionnement relatif ; à titre d'exemple, la jupe 11 est, sur une portion restreinte de sa périphérie, prolongée longitudinalement à l'opposé de la paroi 12, afin d'être reçue dans une encoche complémentaire délimitée à l'extrémité distale de la sonde ; et/ou
- on comprend que les branches 21 et 22 décrites jusqu'ici peuvent être globalement qualifiées de semi-rigides, dans le sens où elles associent une souplesse nécessaire à leur déformation et une rigidité nécessaire à leur passage à travers les parois de la vessie et de l'urètre ; en variantes non représentées, on conçoit donc que, dans certaines de leurs portions, l'une et/ou l'autre de ces branches peuvent être plus souples que rigides ; plus généralement, les branches 21 et 22 sont prévues déformables sans se rompre, aux fins de leur déplacement relatif entre les configurations escamotée et déployée de l'élément de suture 20, étant remarqué que, si besoin, une pièce additionnelle peut être rapportée pour lier fixement l'une à l'autre les extrémités libres des branches, comme il va être expliqué juste ci-après en lien avec un autre mode de réalisation détaillé.

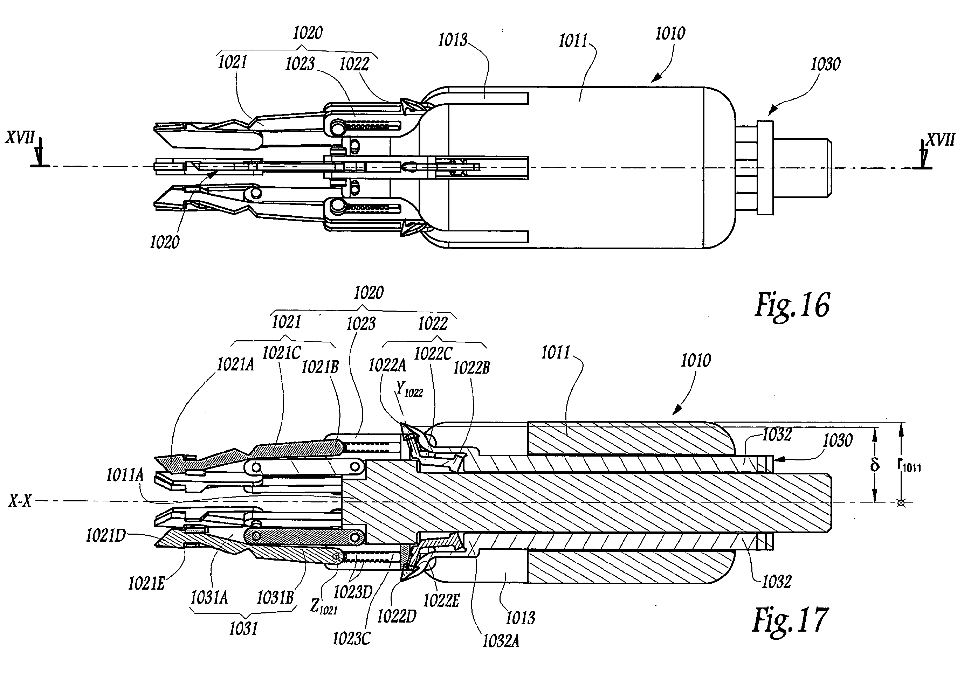

Sur les figures 16 et 17 est représenté un second mode de réalisation d'un système de traitement pour réaliser une anastomose entre la vessie 2 et l'urètre 3. Comme le système 1 décrit jusqu'ici, le système conforme à ce second mode de réalisation comprend des organes de suture 1020, qui, comme détaillé ci-après, sont portés de manière mobile par un corps de support 1010 et sont conçus pour être sollicités en déplacement et en déformation par un mécanisme 1030 porté de manière mobile par le corps de support 1010. Comme décrit plus loin, lors de la présentation détaillée d'un exemple d'utilisation, le système conforme à ce second mode de réalisation comprend également un applicateur 1040 et une sonde 1050.

Comme bien visible sur les figures 16 et 17, le corps 1010 présente une forme allongée, centrée sur un axe longitudinal X-X. Dans l'exemple de réalisation considéré ici, le corps 1010 comporte principalement une tige cylindrique 1011 à base circulaire, centrée sur l'axe X-X.

Comme bien visible sur les figures 17 et 18, chacun des organes de suture 1020 inclut quatre pièces distinctes, qui sont assemblées les unes aux autres, en autorisant, au moins dans certaines configurations de l'organe de suture, une mobilité entre les pièces. Ci-après, trois des quatre pièces précitées vont être décrites plus en détail, en considérant que chaque organe de suture 1020 est dans une configuration initiale vis-à-vis du corps de support 1010, telle que représentée sur les figures 16 et 17. Ainsi, parmi les quatre pièces précitées, chaque organe 1020 inclut deux branches 1021 et 1022 qui sont reliées de manière mobile par un pont 1023 constituant une troisième des quatre pièces précitées. Comme bien visible sur les figures 17 et 18, le pont 1023 présente une forme allongée sensiblement rectiligne, qui s'étend en longueur de manière sensiblement parallèle à l'axe X-X. Chaque pont 1023 est agencé à une extrémité distale étranglée 1011A de la tige 1011 du corps 1010, en étant radialement supporté en direction de l'axe X-X par l'extrémité 1011 A de la tige 1011, tout en étant situé, suivant la périphérie de cette extrémité 1011 A, dans le prolongement axial d'une fente longitudinale 1013 qui est délimitée dans la partie courante de la tige 1011 en débouchant radialement sur l'extérieur de cette partie courante, plus précisément dans la partie d'extrémité distale de cette partie courante, qui forme un épaulement radialement saillant vers l'extérieur vis-à-vis de l'extrémité distale précitée 1011A de la tige 1011. Chaque pont 1023 présente une extrémité longitudinale distale 1023A par rapport à laquelle l'extrémité proximale 1021 B de la branche correspondante 1021 est articulée autour d'un axe de basculement Z₁₀₂₁ qui s'étend de manière sensiblement orthoradiale à l'axe X-X. A son extrémité proximale opposée 1023B, chaque pont 1023 présente un trou traversant ou une gorge, centré sur un axe Y₁₀₂₂ transversal à l'axe X-X : comme représenté sur la figure 17, la partie courante 1022C de la branche 1022 est reçue de manière coulissante dans le trou délimité par l'extrémité proximale 1023B du pont 1023, en y étant orientée selon l'axe Y₁₀₂₂.

Avantageusement, pour des raisons qui apparaîtront plus loin, chaque pont 1023 délimite intérieurement, suivant la longueur de sa partie courante, une fente sensiblement rectiligne 1023C, qui relie les extrémités 1023A et 1023B, et dans l'extrémité distale de laquelle est agencée l'extrémité proximale 1021 B de la branche 1021, articulée autour de l'axe de basculement Z₁₀₂₁. De la sorte, l'extrémité proximale 1021 B de la branche 1021 est à même d'être rapprochée, suivant la direction de l'axe X-X, de l'extrémité proximale 1023B du pont 1023, et ce dans des conditions de fonctionnement qui seront détaillées plus loin, étant remarqué que le retour inverse de l'extrémité 1021 B de la branche 1021 est rendu impossible par des crans anti-retour 1023B dont est intérieurement pourvue la fente 1023C.

A l'opposé de son extrémité 1021 B, chaque branche 1021 présente une extrémité libre 1021A pourvue d'une pointe de perforation 1021D. Avantageusement, pour des raisons qui apparaîtront plus loin, cette pointe 1021 D présente, dans sa zone de liaison avec la partie courante 1021C de la branche 1021, une section transversale plus grande que celle de la partie courante 1021C, de manière à former ainsi un épaulement de transition 1021 E.

Quant à la branche 1022, elle présente, à l'opposé l'une de l'autre et reliées par sa partie courante 1022C, une extrémité proximale 1022B et une extrémité libre 1022A. Cette extrémité libre 1022A présente des aménagements similaires à ceux de l'extrémité libre 1021A de la branche 1021, à savoir une pointe 1022D et un épaulement 1022E de transition avec la partie courante 1022C.

Dans la configuration initiale des figures 16 et 17, la partie courante 1021C de chaque branche 1021 s'étend en longueur globalement suivant la direction de l'axe X-X, ce qui revient à dire que cette partie courante 1021C s'étend sensiblement dans le prolongement rectiligne du pont de liaison 1023. La branche 1022 présente, quant à elle, sa partie courante 1022C coudée, le segment proximal de cette partie courante 1022C s'étendant en longueur de manière sensiblement parallèle à l'axe X-X, en occupant le fond radial d'une des fentes 1013, tandis que le segment distal de la partie courante 1022C est reçu dans le trou ou la gorge, délimité à l'extrémité proximale 1023B du pont de liaison 1023.

De façon similaire à ce qui a été décrit pour le premier mode de réalisation, la configuration initiale, décrite jusqu'ici, des organes de suture 1020 peut être qualifiée de configuration escamotée vis-à-vis du corps 1010, dans le sens où, dans cette configuration, les extrémités libres 1021 A et 1022A des branches 1021 et 1022 de chaque organe 1020 sont, au maximum, distantes radialement de l'axe X-X d'une valeur δ qui est inférieure au rayon extérieur maximal r₁₀₁₁ de la tige 1011 du corps 1010, comme indiqué sur la figure 17. Comme précédemment pour le premier mode de réalisation, cet agencement des organes de suture 1020 permet, comme expliqué ci-après, d'introduire le cops 1010 à l'intérieur d'un orifice d'entrée de la vessie 2, sans interférer avec le bord de cet orifice d'entrée. Bien entendu, les tissus de la vessie 2 peuvent être légèrement étirés si le diamètre du col vésical au repos est trop petit.

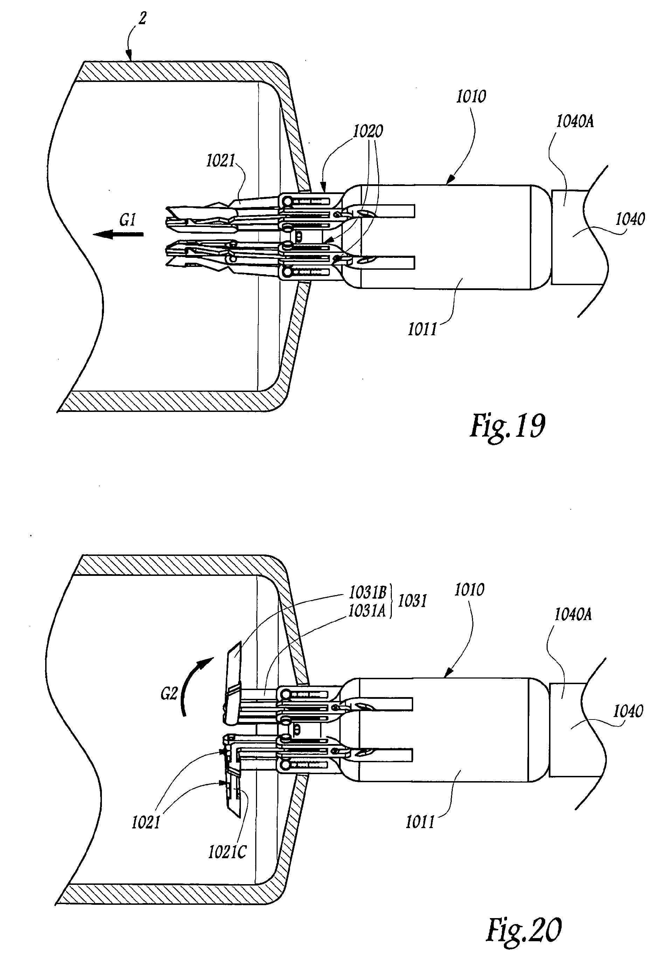

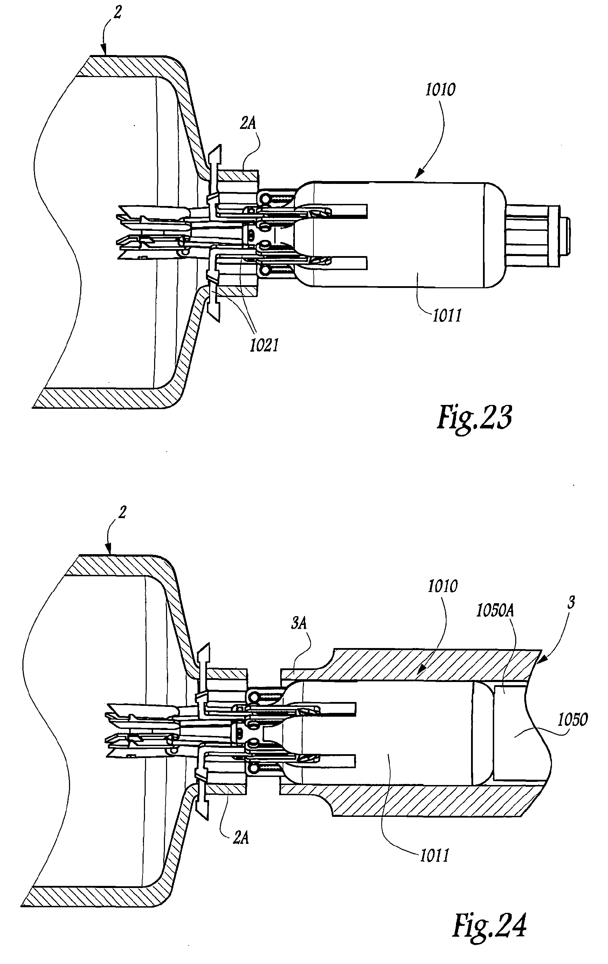

Le mécanisme 1030 est destiné, de manière similaire au mécanisme 30 décrit pour le premier mode de réalisation, à déplacer et déformer les organes de suture 1020 pour les passer de leur configuration initiale escamotée des figures 16 et 17, à une configuration finale déployée, décrite par la suite. Dans l'exemple de réalisation considéré ici, ce mécanisme 1030 comprend divers composants qui vont être décrits ci-après dans le cadre d'un exemple détaillé d'utilisation du système correspondant, en vue de réaliser une anastomose entre la vessie 2, visible sur les figures 19 à 28, et l'urètre 3 visible sur les figures 24 à 28.

Initialement, on considère que le corps 1010, les organes de suture 1020 et le mécanisme 1030 sont mis à la disposition du chirurgien dans leur configuration relative représentée sur les figures 16 et 17. Le corps 1010 est alors manipulé à l'aide de l'applicateur 1040, comme représenté sur la figure 19 : le corps 1010 est rapproché de la vessie 2, jusqu'à introduire au moins l'extrémité distale 1011 A de sa tige 1011 à l'intérieur d'un orifice d'entrée 2B de la vessie 2, en centrant l'axe X-X à l'intérieur de cet orifice d'entrée et en y engageant d'abord les branches 1021 des organes de suture 1020, comme indiqué par la flèche G1 sur la figure 19.

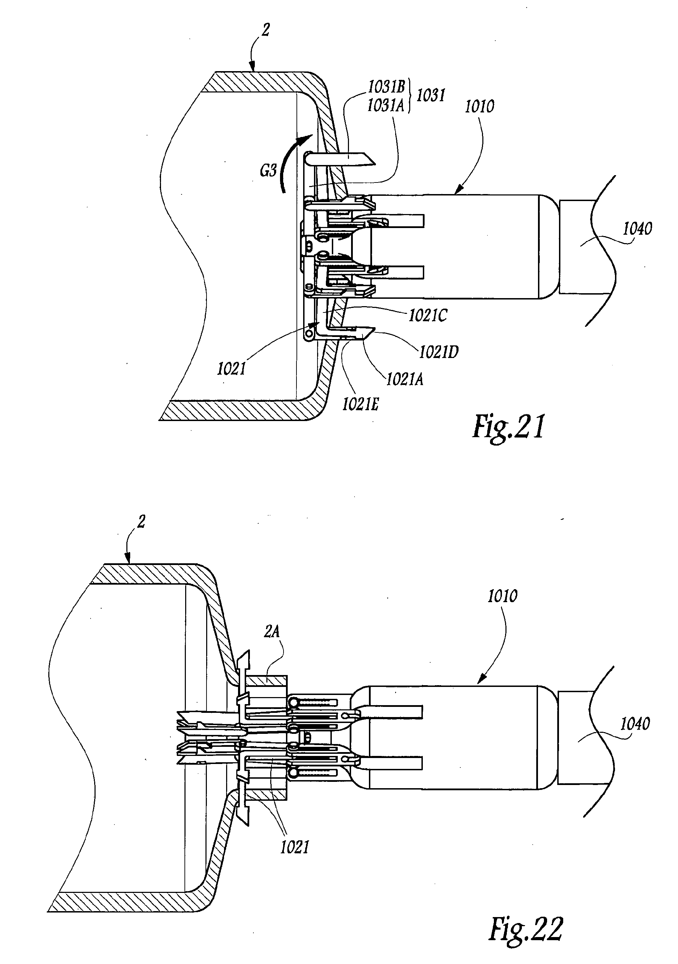

Par le biais d'une commande décrite plus loin, le mécanisme 1030 sollicite alors les branches 1021 des organes de suture 1020 pour passer chacune de ces branches 1020 de sa configuration initiale sensiblement rectiligne de la figue 19, à une configuration déformée en U montrée à la figure 21, en passant par une configuration intermédiaire en L montré à la figure 20. Pour ce faire, le mécanisme 1030 comprend, pour chacune des branches 1021, un bras articulé 1031 qui comprend suivant sa longueur :
- un premier segment rectiligne 1031 A, dont une extrémité longitudinale est articulée sur l'extrémité distale 1011 A de la tige 1011, et
- un second segment rectiligne 1031B dont l'extrémité tournée vers le premier segment 1031 A est articulée sur l'extrémité de ce dernier, opposée à celle articulée sur la tige 1011.

Comme bien visible sur les figures 17 et 18, la partie courante 1021C de chaque branche 1021 court le long des segments 1031A et 1031B, en étant liée de manière amovible à ces segments de sorte que, par basculement du segment 1031 B à 90° par rapport au segment 1031 A, indiqué par la flèche G2 sur la figure 20, la partie courante 1021C de chaque branche 1021 forme un L, comme sur la figure 20, puis, par basculement du segment 1031A à 90 de l'extrémité distale 1011A de la tige 1011, indiqué par la flèche G3 sur la figure 21, la partie courante 1021C est conformée en U, comme montré sur la figure 21. En pratique, eu égard à la présence de la paroi de la vessie 2, délimitant l'orifice d'entrée 2B, les extrémités libres 1021A de chaque branche 1021 traversent de part en part la paroi précitée de la vessie 2, transversalement de l'intérieur vers l'extérieur, lorsque la branche passe de sa forme en L à sa forme en U. La perforation transversale de la paroi précitée de la vessie 2 est réalisée par la pointe 1021 D de chaque extrémité libre 1021 A et/ou par l'extrémité libre, par exemple biseautée à cet effet, du segment 1031 B. Dans tous les cas, une fois que la pointe 1021 D est à l'extérieur de la vessie, l'épaulement 1021 E s'oppose ensuite à ce que la pointe 1021 D retraverse la paroi de la vessie en sens inverse. Ainsi, on comprend que les bras 1021 sont conçus pour guider et contraindre les branches 1021 des organes de suture 1020 pour passer ces organes de suture entre la configuration initiale de la figure 19 et la configuration intermédiaire de la figure 21.

En pratique, les bras 1031 sont entraînés en mouvement par rapport au corps 1010 sous l'action de moyens de commande ad hoc, par l'intermédiaire de l'applicateur 1040. A titre d'exemple, de tels moyens de commande comprennent, voire consistent en des câbles de transmission mécanique, qui s'étendent depuis l'extrémité distale 1040A de l'applicateur 1040, à l'intérieur de la tige 1011 du corps 1010, jusqu'à rejoindre les segments 1031 A et 1031 B de chacun des bras 1031. Plus généralement, il est prévu que l'extrémité distale 1040A de l'applicateur 1040 est conformée pour coopérer avec et ainsi commander une partie du mécanisme 1030, liée cinématiquement aux bras 1031.

Bien entendu, on comprend également que, pour que les branches 1021 passent de leur configuration rectiligne des figures 16 et 17 à leur configuration déformée en U de la figure 21, leur partie courante 1021C est élastiquement déformable ou, plus généralement, déformable sans se rompre. En pratique, les exemples de matériaux biorésorbables, fournis précédemment pour le premier mode de réalisation, peuvent être envisagés pour réaliser la partie courante 1021C des branches 1021, voire, plus globalement, pour réaliser l'intégralité des branches 1021. Plus généralement, dans la mesure où les bras 1031 supportent et guident les branches 1021 dans leur déplacement tout au long de leur mise en oeuvre, on comprend que ces branches 1021 peuvent présenter aussi bien une certaine rigidité, qu'une certaine souplesse, du moment qu'elles se déforment suffisamment en service, sans se rompre.

Suivant une option particulièrement avantageuse, les branches 1021 et le mécanisme 1030 sont conçus pour permettre de tubulariser la paroi de la vessie 2, à travers laquelle les extrémités 1021 A des branches 1021 ont été passées. C'est par exemple le cas avec la forme de réalisation des bras d'entraînement 1031 : en effet, à partir de la configuration de la figure 21 dans laquelle chaque organe de suture présente une forme globale en U, une cinématique dédiée de basculement progressif de chaque bras articulé 1031 permet à l'épaulement 1021E de l'extrémité libre 1021A de chaque branche 1021 d'entraîner la paroi précitée de la vessie 2 jusqu'à lui donner une forme globalement tubulaire, comme sur la figure 22, reconstruisant ainsi facilement et efficacement un col 2A pour la vessie 2. Concomitamment, après libération entre l'extrémité libre 1021A de chaque branche 1021 et le second segment 1031B du bras correspondant 1031, ce dernier est ramené dans sa position initiale, tandis que la partie courante 1021C de la branche 1021 garde sa forme en L. De nouveau, on comprend que les basculements relatifs entre les segments 1031A et 1031B de chaque bras 1031 sont commandés par des moyens ad hoc, par l'intermédiaire de l'applicateur 1040.

Dans un temps opératoire subséquent, l'applicateur 1040 est dégagé, comme représenté sur la figure 23, puis le chirurgien utilise la sonde urétrale 1050, comme représenté sur la figure 24, en particulier dans le cas d'une prostactectomie radicale. Comme décrit en détail plus haut en lien avec le premier mode de réalisation, cette sonde 1050 est introduite dans l'urètre 3 depuis le méat de ce dernier, jusqu'à l'orifice terminal opposé 3A de l'urètre, visible sur la figure 24.

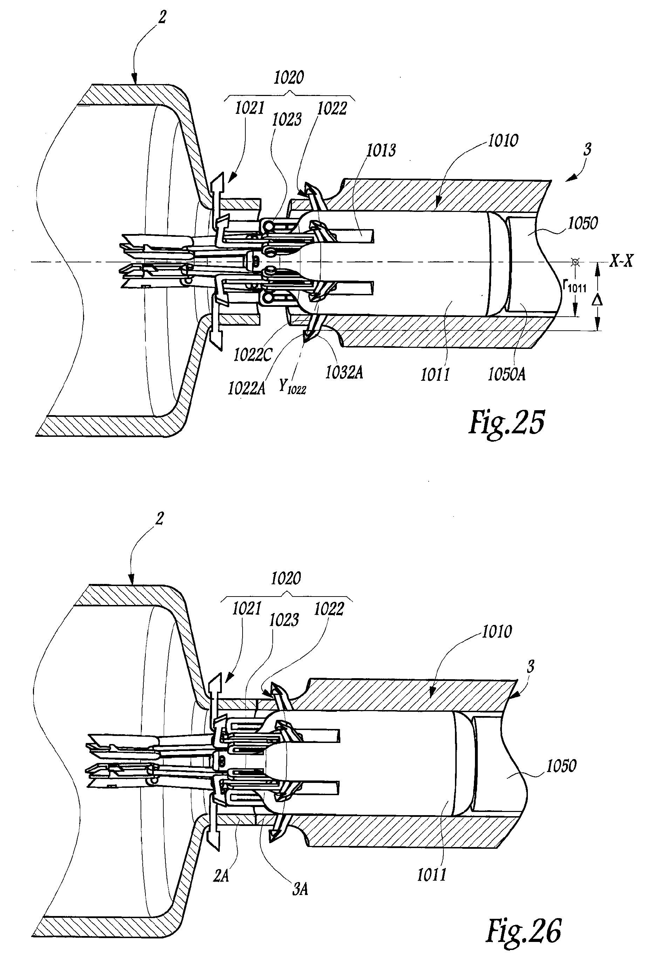

Le chirurgien commande alors, via la sonde urétrale 1050, le déplacement et la déformation des branches 1022 des organes de suture 1020 : chacune de ces branches 1022 passe de sa configuration initiale des figures 16 et 17, à une configuration déployée montrée à la figure 25. En pratique, dans le mode de réalisation considéré ici, chaque branche 1022 est sollicitée en déplacement et déformation par un poussoir dédié 1032, deux de ces poussoirs étant visibles en coupe sur la figure 17 dont les mouvements sont commandés par la sonde 1050, par coopération entre l'extrémité proximale du poussoir et l'extrémité distale 1050A de la sonde. Ainsi, chacun de ces poussoirs 1032 est monté à translation selon la direction de l'axe X-X, en étant reçu de manière coulissante dans des passages complémentaires délimitées au travers, suivant la direction de l'axe X-X, de la tige 1011, chacun des passages précités débouchant, du côté distal, dans l'une des fentes 1013. Moyennant son déplacement en translation selon la direction de l'axe X-X, en direction distale, sous l'action de commande de la sonde 1050, chaque poussoir 1032 entraîne, par son extrémité libre 1032A, l'extrémité proximale 1022B de la branche correspondante 1022 selon un mouvement de translation correspondant. Cependant, eu égard à la résistance du pont correspondant 1023, alors commandée pour être immobile selon la direction de l'axe X-X, la branche 1022 se déforme par effet de rampe. Plus précisément, sa partie courante 1022C passe progressivement à travers le trou ou la gorge, délimité dans l'extrémité proximale 1023B du pont 1023, forçant ainsi le segment longitudinal de cette partie courante 1022C, émergeant de l'extrémité 1023B, à se translater selon la direction de l'axe Y₁₀₂₂, en s'éloignant de la tige 1011. Ainsi, l'extrémité libre 1022A de chaque branche 1022 se translate selon l'axe correspondant Y₁₀₂₂, en s'éloignant du corps 1010. Comme bien visible par comparaison des figures 23 et 24, cette extrémité libre 1022A passe ainsi de l'intérieur à l'extérieur de la paroi délimitant l'orifice 3A de l'urètre 3, la perforation transversale de cette paroi étant réalisée par la pointe correspondante 1022D et/ou par l'extrémité libre 1032A, par exemple prolongée et se terminant par une arête coupante, du poussoir correspondant 1032, tandis que l'épaulement correspondant 1022E empêche ensuite la pointe 1022D de retraverser la paroi précitée en sens inverse. Bien entendu, sur la base de considérations similaires développées plus haut pour la partie courante 1021C des branches 1021, on comprend que la partie courante 1022C des branches 1022 est, elle aussi, réalisée en un matériau déformable, afin de supporter sans se rompre son coudage progressif au travers de l'extrémité 1023B du pont 1023. Plus généralement, dans la mesure où la coopération entre le corps 1010, les poussoirs 1032 et les ponts 1023 immobilisés sur le corps 1010 supportent et guident les branches 1022 dans leur déplacement tout au long de leur mise en oeuvre, on comprend que ces branches 1022 peuvent présenter aussi bien une certaine rigidité qu'une certaine souplesse, du moment qu'elles se déformant suffisamment en service, sans se rompre.

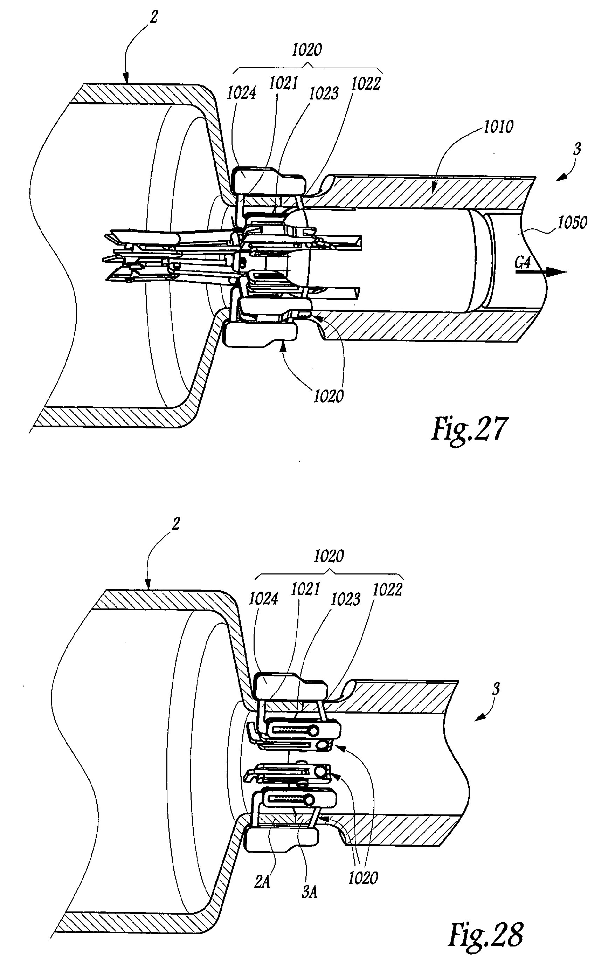

Ainsi, à ce stade de l'intervention, on notera que les extrémités 1021 A, et 1022A des branches 1021 et 1022 se retrouvent distantes radialement de l'axe X-X d'une valeur notée Δ sur la figure 25, qui est supérieure au rayon extérieur r₁₀₁₁ de la jupe 1011 du corps 1010. Ainsi, dans cette configuration des organes de suture 1020, les branches 1021 et 1022 de chacun de ces organes sont prêtes à être liées fixement l'une à l'autre par une pièce rapportées 1024 appartenant à l'organe 1020, cette pièce 1024 étant la quatrième des pièces distinctes formant chaque organe 1020, qui ont été évoquées plus haut. Ces pièces rapportées 1024 sont d'ailleurs mises en place à la figure 27, tandis que l'une d'elle est représentée sur la figure 18, de manière dissociée du reste de l'organe de suture 1020. Cependant, avant de rapporter ces pièces 1024, un aspect optionnel particulièrement avantageux est mis en oeuvre, comme illustré sur la figure 26. Comme bien visible par comparaison entre les figures 25 et 26, les branches 1021 et 1022 de chaque organe de suture 1020 sont rapprochées axialement l'une de l'autre, grâce aux aménagements liés à la fente 1023C, décrits plus haut. En pratique, comme cela a d'ailleurs été évoqué en lien avec le premier mode de réalisation, c'est préférentiellement la vessie 2 qui est sollicitée par le chirurgien pour rapprocher son col 2A de l'orifice 3A de l'urètre 3, ce rapprochement étant accompagné du et/ou commandé par le décalage proximal de l'extrémité 1021B de la branche 1021 le long de la fente 1023C. Sous l'action des crans anti-retour 1023D, le rapprochement relatif des branches 1021 et 1022 est à sens unique, autrement dit irréversible : on peut donc dire que l'accolement jointif entre la berge du col 2A de la vessie 2 et la berge de l'orifice 3A de l'urètre 3 est serré dans la direction de l'axe X-X, autrement dit dans la direction longitudinale de l'urètre.

Comme évoqué rapidement plus haut, le temps opératoire suivant consiste à rapporter les pièces 1024, comme représenté sur la figure 27. En pratique, chaque pièce rapportée 1024 est adaptée pour coopérer par complémentarité de formes avec, à la fois, l'extrémité libre 1021 A de la branche correspondante 1021 et l'extrémité libre 1022A de la branche correspondante 1022, de manière à se trouver liées fixement à ces extrémités 1021A et 1022A, ces dernières se trouvant ainsi liées fixement l'une à l'autre par la pièce 1024. Diverses formes de réalisation sont envisageables pour les pièces 1024 du moment que ces diverses formes de réalisation permettent de clipser fixement les extrémités 1021A et 1022A une fois que ces extrémités sont dans la configuration déployée des figures 25 et 26. A ce propos, les épaulements 1021 E et 1022E des extrémités 1021 A et 1022A des branches sont avantageusement utilisés pour coopérer par complémentarité de formes avec des zones dédiées de la pièce 1024, afin d'empêcher le dégagement de ces extrémités une fois qu'elles sont mises en prise avec la pièce 1024.

De la même façon, diverses techniques opératoires peuvent être envisagées pour mettre en place les pièces 1024: ces pièces 1024 peuvent être individuellement rapportées à la main par le chirurgien, ou bien être portées par un ancillaire ad hoc, ou bien être supportées par un ancillaire précédemment utilisé lors de l'intervention chirurgicale, notamment par l'applicateur 1040. De surcroît, le moment où les pièces 1024 sont mises en place, peut être différent que celui considéré en regard à la figure 27 : en effet, à titre de variante non représentée, il peut être envisagé de rapporter les pièces 1024 dès que les extrémités 1021 A des branches 1021 ont traversé la paroi de la vessie 2, autrement dit dès l'étape illustrée à la figure 21, étant entendu que les extrémités 1022A des branches 1022 ne sont ensuite clipser aux pièces 1024 déjà présentes qu'une fois que ces extrémités 1022A ont traversé l'urètre 3, c'est-à-dire à partir de l'étape illustrée à la figure 25. D'ailleurs, dans le prolongement de cette variante opératoire, les pièces 1024 peuvent avantageusement être utilisées pour former des contre-appuis respectifs pour les branches 1021 lorsque la pointe 1021 B de ces dernières perfore la paroi de la vessie 2.

Il reste alors au chirurgien à finir de dégager les organes de suture 1020 vis-à-vis du corps 1010. Pour ce faire, le cas échéant après avoir actionné une commande de libération mécanique, via notamment la sonde urétrale 1050, le corps 1010 étant évacué par l'intérieure de l'urètre 3, par entraînement de la sonde urétrale 1050, comme indiqué par la flèche G4 sur la figure 27. L'intervention chirurgicale prend alors fin, les organes de suture 1020 restant en place dans leur configuration déployée, qui est figée par les pièces rapportées 1024, en chevauchant chacun l'interface d'anastomose réalisée entre le col 2A de la vessie 2 et l'orifice terminal 3A de l'urètre 3, comme représenté à la figure 28.

Par la suite, les organes de suture 1020 vont être progressivement résorbés, après cicatrisation de l'anastomose.

Ainsi, on comprend de la description qui précède du second mode de réalisation, en lien avec les figures 16 à 28, que ce second mode de réalisation diffère du premier mode de réalisation par plusieurs aspects, à savoir, entre autres, les aspects suivants :
- plutôt que d'être d'un seul tenant comme les éléments de suture 20, les organes de suture 1020 sont constitués chacun des quatre pièces distinctes 1021, 1022, 1023 et 1024, chaque organe de suture 1020 passant, comme chaque élément de suture 20, d'une configuration initiale escamotée, dans laquelle les extrémités libres 1021A et 1022A des branches 1021 et 1022 sont plus près radialement de l'axe X-X que ne l'est la surface extérieure du corps 1010, notamment à des fins d'insertion sans interférence et de mise en place de ce corps à l'intérieur de la vessie, à une configuration finale déployée, dans laquelle les extrémités libres 1021 A et 1022A des branches 1021 et 1022 se retrouvent liées fixement l'une à l'autre par une des pièces rapportées 1024, le passage entre ces configurations escamotée et déployée étant réalisé, comme pour les éléments de suture 20, moyennant la déformation au moins locale et le déplacement des branches 1021 et 1022 ;
- de plus, grâce à la réalisation du pont 1023 sous forme d'une pièce distincte, à laquelle sont assemblées de manière mobile les branches 1021 et 1022, le second mode de réalisation permet un serrage significatif de l'anastomose, ce serrage pouvant être d'ailleurs global ou adaptatif, c'est-à-dire que, dans ce dernier cas, les contraintes de serrage respectivement appliquées aux organes de suture sont propres à chaque organe et éventuellement différentes les unes des autres ;
- plutôt que la totalité du mécanisme de sollicitation en déplacement et en déformation des organes de suture soit commandée par la sonde urètrale, comme c'est le cas pour le mécanisme 30, le mécanisme 1030 est commandé, pour partie, par l'applicateur 1040 et, pour une autre partie, par la sonde urétrale 1050 ; un des avantages liés à cet aspect est que les branches 1021 permettent, alors que le corps 1010 est encore lié mécaniquement à l'applicateur 1040, d'immobiliser temporairement le corps 1010 dans la vessie à traiter 2 ; autrement dit, en plus d'assurer la fonction des branches 21, les branches 1021 assurent au moins partiellement la fonction dévolue aux crochets 14 ; et
- comme expliqué plus haut, le mécanisme 1030 assure, en plus d'une fonction similaire à celle du mécanisme 30, à savoir solliciter en déplacement et déformation les organes de suture, une fonction de tubularisation vis-à-vis de la vessie 2, permettant ainsi de reconstruire facilement le col 2A de cette vessie.

Bien entendu, divers aménagements et variantes au système décrit en regard des figures 16 à 28, ainsi qu'à la méthode de mise en oeuvre de ce système sont par ailleurs envisageables. En particulier, des variantes à ce système consistent à ce que seuls certains des aspects différenciant vis-à-vis du premier mode de réalisation soient présents.

Par ailleurs, les considérations étrangères aux spécifications développées dans le présent document ne sont pas limitatives pour la présente invention. A titre d'exemple, les différents composants du système, autres que les organes de suture biorésorbables, sont indifféremment jetables ou ré-utilisables.

Enfin, bien que toute la description qui précède a été faite dans le contexte de la réalisation d'une anastomose entre la vessie 2 et l'urètre 3 d'un patient, on comprend que le système selon l'invention, ainsi que sa méthode de mise en oeuvre, peuvent être plus généralement appliqués à deux conduits creux d'un patient, nécessitant d'être reliés par une anastomose. Ainsi, outre l'exemple de la vessie et de l'urètre, les deux conduits creux précités peuvent consister en des conduits intestinaux, vasculaires, etc.

## Revendications

1. Système de traitement chirurgical pour réaliser une anastomose entre deux conduits creux (2, 3) d'un patient, tels que la vessie (2) et l'urètre (3) du patient, notamment après une prostatectomie, ce système comprenant :
- au moins un organe de suture (20 ; 1020), réalisé en un matériau bio-résorbable et incluant deux branches (21, 22 ; 1021, 1022), qui présentent des extrémités libres respectives (21A, 22A ; 1021A, 1022A) et qui, en dehors desdites extrémités libres, sont au moins en partie déformables,
- un corps de support (10 ; 1010) allongé, qui est adapté, à la fois, pour porter de manière mobile le ou les organes de suture (20 ; 1020) et pour être introduit suivant la direction de son axe longitudinal central (X-X) dans l'un des deux conduits creux (2, 3) du patient, et
- un mécanisme (30; 1030) de sollicitation du ou des organes de suture (20; 1020), qui est porté de manière mobile par le corps de support (10 ; 1010), en étant au moins en partie agencé dans le corps de support, et qui est adapté pour déplacer et déformer le ou chaque organe de suture entre une configuration escamotée, dans laquelle les extrémités libres respectives (21A, 22A ; 1021A, 1022A) des deux branches (21, 22) de l'organe de suture sont déplaçables l'une par rapport à l'autre, en étant disposées chacune à une distance radiale (δ) de l'axe (X-X), qui est inférieure au rayon extérieur (r₁₁) du corps de support et une configuration déployée, dans laquelle les extrémités libres respectives des deux branches de l'organe de suture sont disposées à l'extérieur du corps de support en étant situées chacune à une distance radiale (Δ) de l'axe, qui est supérieure au rayon extérieur du corps de support,
**caractérisé en ce que** les extrémités libres respectives (21A, 22A; 1021A, 1022A) des deux branches (21, 22 ; 1021, 2022) sont adaptées pour être liées fixement l'une à l'autre en coopérant par complémentarité de formes soit l'une avec l'autre, soit avec une pièce rapportée (1024) de l'organe de suture,
et **en ce que**, dans la configuration déployée, les extrémités libres respectives des deux branches sont disposées pour être liées fixement l'une à l'autre, après avoir traversé transversalement de l'intérieur vers l'extérieur les parois respectives des deux conduits creux (2, 3) du patient.

2. Système suivant la revendication 1, **caractérisé en ce que** l'extrémité libre (1021A ; 1022A) d'une ou de chacune des deux branches (1021 ; 1022) du ou de chaque organe de suture (1020) présente, à l'opposé du reste de la branche, une pointe (1021D ; 1022D), avantageusement à même de perforer la paroi des conduits creux (2, 3), laquelle pointe présente, dans sa zone de liaison avec le reste de la branche, une section transversale plus grande que celle du reste de la branche de manière à former un épaulement de transition (1021E; 1022E) qui est adapté pour empêcher le dégagement de l'extrémité libre de la branche vis-à-vis de ladite paroi, ainsi que vis-à-vis de la pièce rapportée (1024) une fois que cette extrémité libre est mise en prise avec cette pièce rapportée.

3. Système suivant l'une des revendications 1 ou 2, **caractérisé en ce que** le système comprend en outre un applicateur (40 ; 1040) de mise en place et de fixation du corps de support (10) dans l'un des conduits creux (2, 3).

4. Système suivant l'une des revendications précédentes, **caractérisé en ce que** le mécanisme (1030) et/ou l'applicateur (40) sont pourvus de moyens (1031 B ; 1032A; 42) pour affaiblir, voire perforer la paroi dudit conduit creux (2, 3) en une ou plusieurs portions destinées à être traversées par le ou les organes de suture (20) lorsqu'ils passent de leur configuration escamotée à leur configuration déployée.

5. Système suivant l'une quelconque des revendications précédentes, **caractérisé en ce que** le corps de support (10) est pourvu de moyens (14) d'immobilisation de manière amovible dans l'un des conduits creux (2, 3), ces moyens d'immobilisation étant notamment commandés par l'applicateur (40).

6. Système suivant l'une des revendications 3 à 5, **caractérisé en ce que** l'applicateur (1040) présente une extrémité distale (1040A) conformée pour coopérer avec et ainsi commander au moins une partie du mécanisme (1030), par exemple via au moins un câble de transmission.

7. Système suivant l'une quelconque des revendications précédentes, **caractérisé en ce que** le système comprend en outre une sonde (50 ; 1050) de commande du mécanisme (30 ; 1030).

8. Système suivant la revendication 7, **caractérisé en ce que** la sonde (50 ; 1050) est adaptée pour être introduite dans l'un des conduits (2, 3), notamment l'urètre (3) depuis le méat jusqu'à l'orifice terminal (3A) de l'urètre, et présente une extrémité distale (50A ; 1050A) conformée pour coopérer avec et ainsi commander au moins une partie du mécanisme (30 ; 1030).

9. Système suivant l'une quelconque des revendications précédentes, **caractérisé en ce que** le mécanisme (30 ; 1030) est adapté pour déplacer et déformer le ou chaque organe de suture (20 ; 1020) entre les configurations escamotée et déployée en passant par l'une puis l'autre de deux configurations intermédiaires, à savoir une première configuration intermédiaire, dans laquelle une première (21 ; 1021) des deux branches (21, 22 ; 1021, 1022) de l'organe de suture s'étend en travers de la paroi d'un des deux conduits creux (2, 3) du patient, avec son extrémité libre (21A; 1021A) qui est située à l'extérieur de cette paroi et qui n'est pas liée fixement à l'extrémité libre (22A ; 1022A) de la seconde branche (22 ; 1022), et une seconde configuration intermédiaire, dans laquelle la seconde branche (22 ; 1022) s'étend en travers de la paroi de l'autre dans deux conduits creux, avec son extrémité libre (22A ; 1022A) qui est située à l'extérieur de cette paroi et qui n'est pas liée fixement à l'extrémité libre (21A; 1021A) de la première branche (21 ; 1021).

10. Système suivant la revendication 9, **caractérisé en ce que** le mécanisme (30) comporte :
- une tirette (31) qui, par rapport au corps du support (10), est montée en translation selon l'axe longitudinal (X-X) et qui est adaptée pour entraîner en translation le ou chaque organe de suture (20) pour le passer de sa configuration escamotée à sa première configuration intermédiaire ;
- un poussoir (32), qui est monté à translation selon l'axe (X-X) par rapport à la tirette (31) et qui est adapté pour déformer, notamment par effet de rampe, la seconde branche (22) du ou de chaque organe de suture (20) pour passer cet organe de suture de sa première configuration intermédiaire à sa seconde configuration intermédiaire, et
- un guide (33), qui est lié fixement au corps de support (10) suivant l'axe (X-X) et qui, pour le ou chaque organe de suture (20), inclut une portion d'écartement (332) interposée entre les deux branches (21, 22) de l'organe de suture et délimitant à la fois une surface de rampe (332B) pour la première branche (21) et une surface d'immobilisation radiale (332A) pour la seconde branche (22) de telle sorte que, par translation (F2) de la tirette (31), l'extrémité libre (21A) de la première branche (21) s'écarte, suivant une direction sensiblement radiale à l'axe, vis-à-vis de l'extrémité libre (22A) de la seconde branche (22) lorsque l'organe de suture passe de sa configuration escamotée à sa première configuration intermédiaire, puis, par translation opposée (F3) du poussoir (32), l'extrémité libre (22A) de la seconde branche (22) se rapproche, suivant une direction sensiblement radiale à l'axe, vis-à-vis de l'extrémité libre (21A) de la première branche (21) lorsque l'organe de suture passe de sa première configuration intermédiaire à sa seconde configuration intermédiaire.

11. Système suivant la revendication 10, **caractérisé en ce que** le guide (33) est monté à rotation autour de l'axe (X-X) par rapport au corps de support (10) et est adapté pour, alors que le ou chaque organe de suture (20) est dans sa seconde configuration intermédiaire, être entraîné en rotation sur lui-même de manière à dégager, suivant une direction orthoradiale à l'axe, sa portion d'écartement (332) d'entre les deux branches (21, 22) de l'organe de suture pour passer cet organe de suture dans sa configuration déployée.

12. Système suivant la revendication 11, **caractérisé en ce que**, pour le ou chaque organe de suture (20), la portion d'écartement (332) du guide (33) est pourvu d'un talon (334), qui prolonge, suivant une direction périphérique à l'axe (X-X), la surface d'immobilisation radiale (332A) pour la seconde branche (22) et qui est relié à la surface de rampe (332B) par un épaulement en creux (335) dans lequel la première branche (21) est reçue par retour élastique pour engager mécaniquement l'extrémité libre (21 A) de cette première branche avec l'extrémité libre (22A) de la seconde branche (22).

13. Système suivant l'une quelconque des revendications 10 à 12, **caractérisé en ce que** la tirette (31) est montée à rotation autour de l'axe (X-X) par rapport au corps de support (10) et est adaptée pour, pendant que le ou chaque organe de suture (20) passe de sa seconde configuration intermédiaire à sa configuration déployée, déplacer radialement vers l'extérieur du corps de support, par effet de came, l'extrémité (21 B) de la première branche (21) de l'organe de suture, opposée à son extrémité libre (21A).

14. Système suivant ta revendication 9, **caractérisé en ce que** le mécanisme (1030) comporte:
- pour chaque organe de suture (1020), un bras (1031) qui est monté basculant sur le corps de support (1010) et le long d'au moins une partie duquel s'étend la première branche (1021) de l'organe de suture (1020) de manière que le bras contraint et guide en déplacement cette première branche pour passer l'organe de suture de sa configuration escamotée à sa première configuration intermédiaire ; et
- au moins un poussoir (1032), qui est monté à translation selon l'axe (X-X) par rapport au corps de support (1010) et qui est adapté pour agir sur la seconde branche (1022) du ou des organes de suture (1020) et la contraindre à se déformer par rapport au reste de l'organe de suture, notamment par effet de rampe, pour passer chaque organe de suture de sa première configuration intermédiaire à sa seconde configuration intermédiaire.

15. Système suivant l'une quelconque des revendications 9 à 14, **caractérisé en ce que** la première branche (1021) du ou de chaque organe de suture (1020) s'étend le long d'une partie du mécanisme (1030), en particulier le long de deux segments (1031A, 1031 B) qu'inclut le bras (1031) et de qui sont articulés l'un à l'autre, manière que, en particulier par basculement relatif des deux segments précités, la première branche de l'organe de suture dans sa première configuration intermédiaire présente successivement une forme globale de U et une forme globale de L pour tubulariser la paroi du conduit creux (2) à travers laquelle la première branche s'étend.

16. Système suivant l'une quelconque des revendications précédentes, **caractérisé en ce que** le ou chaque organe de suture (1020) inclut, en outre, un pont (1023) de liaison mobile entre les deux branches (1021, 1022), pont auquel l'une et/ou l'autre des deux branches sont assemblées de manière mobile, en particulier pont auquel la première branche (1021) de l'organe de suture est assemblée de manière mobile, la première branche étant déplacée par rapport au pont lorsque l'organe de suture passe de sa configuration escamotée à sa première configuration intermédiaire, et/ou pont auquel la seconde branche (1022) de l'organe de suture est assemblé de manière mobile, la seconde branche étant déplacée par rapport au pont lorsque l'organe de suture passe de sa première configuration intermédiaire à sa seconde configuration intermédiaire.

17. Système suivant la revendication 16, **caractérisé en ce que** le pont (1023) est pourvu de moyens (1023C, 1023D) de rapprochement axial des deux branches (1021, 1022) du ou de chaque organe de suture (1020), ces moyens de rapprochement étant adaptés pour, lorsque l'organe de suture est dans sa configuration déployée, guider axialement l'une vers l'autre les deux branches, notamment à l'endroit où elles traversent les parois des conduits, tout en retenant les deux branches l'une vis-à-vis de l'autre pour empêcher qu'elles s'écartent axialement, de manière à serrer l'anastomose entre les conduits.

18. Système suivant l'une quelconque des revendications 1 à 15, **caractérisé en ce que** les deux branches (21, 22) du ou de chaque organe de suture (20) sont, à l'opposé de leur extrémité libre (21A, 22A), liées à demeure l'une à l'autre de manière élastiquement déformable de sorte que l'organe de suture forme un élément de suture (20), notamment d'un seul tenant.

## Patentansprüche

1. System zur chirurgischen Behandlung zum Realisieren einer Anastomose zwischen zwei hohlen Gängen (2, 3) eines Patienten, wie die Harnblase (2) und die Harnröhre (3) des Patienten, insbesondere nach einer Prostatektomie, wobei dieses System Folgendes umfasst:
- mindestens eine Nahteinrichtung (20, 1020), das aus einem bioresorbierbaren Material hergestellt wurde und zwei Bügel (21, 22; 1021, 1022) umfasst, die jeweilige freie Enden (21A, 22A; 1021A, 1022A) aufweisen, und die, außerhalb der freien Enden, zumindest zum Teil verformbar sind,
- einen langgestreckten Trägerkörper (10; 1010), der angepasst ist, um gleichzeitig, die Nahteinrichtung(en) (20, 1020) auf bewegliche Weise zu tragen und um in der Richtung seiner Längsmittelachse (X-X) in einen der zwei hohlen Gänge (2, 3) des Patienten eingeführt zu werden, und
- einen Mechanismus (30; 1030) zum Beaufschlagen der Nahteinrichtung(en) (20; 1020), die auf bewegliche Weise durch den Trägerkörper (10; 1010) getragen wird, indem er zumindest zum Teil in dem Trägerkörper ausgebildet ist, und der angepasst ist, um die oder jede Nahteinrichtung zu bewegen und zu verformen zwischen einer eingefahrenen Anordnung, in der die jeweiligen freien Enden (21A, 22A; 1021A, 1022A) der zwei Bügel (21, 22) der Nahteinrichtung relativ zueinander beweglich sind, indem sie in einem radialen Abstand (5) von der Achse (X-X) angeordnet sind, der kleiner ist als der Außenradius (r₁₁) des Trägerkörpers, und einer ausgefahrenen Anordnung, in der die jeweiligen freien Enden der zwei Bügel der Nahteinrichtung außerhalb des Trägerkörpers angeordnet sind, indem sie sich jeweils in einem radialen Abstand (Δ) von der Achse befinden, der größer ist als der Außenradius des Trägerkörpers,
**dadurch gekennzeichnet, dass** die jeweiligen freien Enden (21A, 22A; 1021A, 1022A) der zwei Bügel (21, 22; 1021, 1022) angepasst sind, um fest miteinander verbunden zu werden, indem sie durch Formschlüssigkeit entweder miteinander oder mit einem angesetzten Teil (1024) der Nahteinrichtung zusammenwirken,und dass, in der ausgefahrenen Anordnung, die jeweiligen freien Enden der zwei Bügel angeordnet sind, um fest miteinander verbunden zu werden, nachdem sie quergerichtet von innen nach außen die jeweiligen Wände der zwei hohlen Gänge (2, 3) des Patienten durchquert haben.

2. System nach Anspruch 1, **dadurch gekennzeichnet, dass** das freie Ende (1021A; 1022A) eines oder jedes der zwei Bügel (1021; 1022) der oder jeder Nahteinrichtung (1020), dem übrigen Bügel entgegengesetzt, eine Spitze (1021D; 1022D) aufweiset, die vorteilhafterweise in der Lage ist, die Wand der hohlen Gänge (2, 3) zu perforieren, wobei die Spitze in ihrer Tone zur Verbindung mit dem übrigen Bügel einen Querschnitt aufweist, der größer als der des übrigen Bügels ist, derart, dass sie einen Übergangsabsatz (1021E; 1022E) bildet, der angepasst ist, um die Freigabe des freien Endes des Bügels gegenüber der Wand sowie gegenüber dem angesetzten Teil 1024) zu verhindern, sobald dieses freie Ende in Eingriff mit diesem angesetzten Teil gebracht ist.

3. System nach einem der Ansprüche 1 oder 2, **dadurch gekennzeichnet, dass** das System ferner einen Applikator (40; 1040) zum Positionieren und Befestigen des Trägerkörpers (10) in einem der hohlen Gänge (2, 3) umfasst.

4. System nach einem der vorhergehenden Ansprüche, **dadurch gekennzeichnet, dass** der Mechanismus (1030) und/oder der Applikator (40) mit Mitteln (1031B; 1032A; 42) versehen sind, um die Wand des hohlen Gangs (2, 3) in einem oder mehreren Abschnitten, die dazu bestimmt, von der oder den Nahteinrichtung(en) (20) durchquert zu werden, wenn sie aus ihrer eingefahrenen Anordnung in ihre ausgefahrene Anordnung übergehen, in ihrer Festigkeit zu beeinträchtigen oder sogar zu perforieren.

5. System nach einem der vorhergehenden Ansprüche, **dadurch gekennzeichnet, dass** der Trägerkörper (10) mit Mitteln (14) zum lösbaren Festlegen in einem der hohlen Gänge (2, 3) versehen ist, wobei diese Festlegungsmittel insbesondere durch den Applikator (40) gesteuert werden.

6. System nach einem der Ansprüche 3 bis 5, **dadurch gekennzeichnet, dass** der Applikator (1040) ein distales Ende (1040A) aufweist, das für das Zusammenwirken mit und dadurch das Steuern mindestens eines Teils des Mechanismus (1030), zum Beispiel über ein Übertragungskabel, ausgebildet ist.

7. System nach einem der vorhergehenden Ansprüche, **dadurch gekennzeichnet, dass** das System ferner eine Sonde (50; 1050) zum Steuern des Mechanismus (30; 1030) umfasst.

8. System nach Anspruch 7, **dadurch gekennzeichnet, dass** die Sonde (50; 1050) angepasst ist, um in einen der hohlen Gänge (2, 3), insbesondere die Harnröhre (3) von der Mündung bis zur Endöffnung (3A) der Harnröhre, eingeführt zu werden, und ein distales Ende (50A; 1050A) aufweist, das für das Zusammenwirken mit und dadurch das Steuern mindestens eines Teils des Mechanismus (30; 1030) ausbebildet ist.

9. System nach einem der vorhergehenden Ansprüche, dadurch gekenntzeichnet, dass der Mechanismus (30; 1030) angepasst ist, um die oder jede Nahteinrichtung (20; 1020) zwischen der eingefahrenen und ausgefahrenen Unordnung zu bewegten und zu verformten, indem es eine und dann die andere von zwei Zwischenanordnungen durchläuft, das heißt, eine erste Zwischenanordnung, in der sich ein erster (21; 1021) der zwei Bügel (21, 22; 1021, 1022) der Nahteinrichtung durch die Wand eines der zwei hohlen Gänge (2, 3) des Patienten hindurch erstreckt, wobei sich sein freies Ende (21A; 1021A) außerhalb dieser Wand befindet und mit dem freien Ende (22A; 1022A) des zweiten Bügels (22; 1022) nicht fest verbunden ist, und eine zweite Zwischenanordnung, in der sich der zweite (22; 1022) durch die Wand des anderen der zwei hohlen Gänge hindurch erstreckt, wobei sich sein freies Ende (22A; 1022A) außerhalb dieser Wand befindet und mit dem freien Ende (21A; 1021A) des ersten Bügels (21; 1021) nicht fest verbunden ist.

10. System nach Anspruch 9, **dadurch gekennzeichnet, dass** der Mechanismus (30) Folgendes aufweist:
- eine Zugschnur (31), die relativ zu dem Trägerkörper (10) entlang der Längsachse (X-X) verschiebbar gelagert ist und die angepasst ist, um die oder jede Nahteinrichtung (20) zur Verschiebung anzutreiben, um sie aus ihrer eingefahrenen Anordnung in ihre erste Zwischenanordnung zu bringen;
- ein Schubelement (32), das relativ zu der Zugschnur (31) entlang der Achse (X-X) verschiebbar gelagert ist und das angepasst ist, um insbesondere durch Rampenwirkung den zweiten Bügel (22) der oder jeder Nahteinrichtung (20) zu verformen, um diese Nahteinrichtung aus ihrer ersten Zwischenanordnung in ihre zweite Zwischenanordnung zu bringen, und
- eine Führung (33), die mit dem Trägerkörper (10) entlang der Achse (X-X) fest verbunden ist und die, für die oder jede Nahteinrichtung (20), einen Abstandsabschnitt (332) umfasst, der zwischen den zwei Bügeln (21, 22) der Nahteinrichtung eingebracht ist und gleichzeitig eine Rampenfläche (332B) für den ersten Bügel (21) und eine radiale Festlegungsfläche (332A) für den zweiten Bügel (22) begrenzt, derart, dass sich durch Verschiebung (F2) der Zugschnur (31) das freie Ende (21A) des ersten Bügels (21) in einer zu der Achse im Wesentlichen radialen Richtung gegenüber dem freien Ende (22A) des zweiten Bügels (22) beabstandet, wenn die Nahteinrichtung aus ihrer eingefahrenen Anordnung in ihre erste Zwischenanordnung übergeht, und sich durch entgegengesetzte Verschiebung (F3) des Schubelements (32) das freie Ende (22A) des zweiten Bügels (22), in einer zu der Achse im Wesentlichen radialen Richtung gegenüber dem freien Ende (21A) des ersten Bügels (21) annähert, wenn die Nahteinrichtung aus ihrer ersten Zwischenanordnung in ihre zweite Zwischenanordnung übergeht.

11. System nach Anspruch 10, **dadurch gekennzeichnet, dass** die Führung (33) relativ zu dem Trägerkörper (10) um die Achse (X-X) drehbar gelagert ist und angepasst ist, um während die oder jede Nahteinrichtung (20) in ihrer zweiten Zwischenanordnung ist zur Drehung um sich selbst angetrieben zu werden, derart, dass sie ihren Abstandsabschnitt (332) zwischen den zwei Bügeln (21, 22) der Nahteinrichtung in zu der Achse orthoradialer Richtung freigibt, um diese Nahteinrichtung in ihre ausgefahrene Anordnung zu bringen.

12. System nach Anspruch 11, **dadurch gekennzeichnet, dass**, für die oder jede Nahteinrichtung (20) der Abstandsabschnitt (332) der Führung (33) mit einem Absatz (334) versehen ist, der entlang einer zu der Achse (X-X) umfänglichen Richtung die radiale Festlegungsfläche (332A) für den zweiten Bügel (22) fortsetzt und der mit der Rampenfläche (332A) durch einen vertieften Absatz (335) verbunden ist, in dem der erste Bügel (21) federnd aufgenommen ist, um das freie Ende (21A) dieses ersten Bügels mit dem freien Ende (22A) des zweiten Bügels (22) mechanisch in Eingriff zu bringen.

13. System nach einem der Ansprüche 10 bis 12, **dadurch gekennzeichnet, dass** die Zugschnur (31) relativ zu dem Trägerkörper (10) um die Achse (X-X) drehbar gelagert ist und angepasst ist, um während die oder jede Nahteinrichtung (20) aus ihrer zweiten Zwischenanordnung in ihre ausgefahrene Anordnung übergeht, das Ende (21B) des ersten Bügels (21) der Nahteinrichtung, das seinem freien Ende (21A) gegenüberliegt, durch Nockenwirkung in radialer Richtung nach außerhalb des Trägerkörpers zu bewegen.

14. System nach Anspruch 9, **dadurch gekennzeichnet, dass** der Mechanismus (1030) Folgendes aufweist:
- für jede Nahteinrichtung (1020) einen Arm (1031), der an dem Trägerkörper (1010) schwenkbar gelagert ist und entlang mindestens eines Teils von diesem sich der erste Bügel (1021) der Nahteinrichtung (1020) erstreckt, derart, dass der Arm diesen ersten Bügel zwingt und in der Bewegung führt, um die Nahteinrichtung aus ihrer eingefahrenen Anordnung in seine erste Zwischenanordnung zu bringen; und
- mindestens ein Schubelement (1032), das relativ zu dem Trägerkörper (1010) entlang der Achse (X-X) verschiebbar gelagert ist und das angepasst ist, um auf den zweiten Bügel (1022) der Nahteinrichtung(en) (1020) einzuwirken und ihn dazu zu zwingen, sich relativ zu der übrigen Nahteinrichtung, insbesondere durch Rampenwirkung, zu verformen, um jede Nahteinrichtung aus ihrer ersten Zwischenanordnung in ihre zweite Zwischenanordnung zu bringen.

15. System nach einem der Ansprüche 9 bis 14, **dadurch gekennzeichnet, dass** sich der erste Bügel (1021) der oder jeder Nahteinrichtung (1020) entlang eines Teils des Mechanismus (1030) erstreckt, insbesondere entlang von zwei Segmenten (1031A, 1031B), die der Arm (1031) umfasst und die aneinander angelenkt sind, derart, dass, insbesondere durch relatives Verschwenken der zwei oben genannten Segmente der erste Bügel der Nahteinrichtung in ihrer ersten Zwischenanordnung nacheinander eine allgemeine U-Form und eine allgemeine L-Form aufweist, um die Wand des hohlen Gangs (2), durch die hindurch sich der erste Bügel erstreckt, zu tubularisieren.

16. System nach einem der vorhergehenden Ansprüche, **dadurch gekennzeichnet, dass** die oder jede Nahteinrichtung (1020) ferner eine Brücke (1023) zur beweglichen Verbindung zwischen den zwei Bügeln (1021, 1022) umfasst, wobei mit dieser Brücke der eine und/oder der andere der zwei Bügel beweglich verbunden sind, wobei mit dieser Brücke insbesondere der erste Bügel (1021) der Nahteinrichtung beweglich verbunden ist, wobei der erste Bügel relativ zu der Brücke bewegt wird, wenn die Nahteinrichtung aus ihrer eingefahrenen Anordnung in ihre erste Zwischenanordnung übergeht, und/oder wobei mit dieser Brücke der zweite Bügel (1022) der Nahteinrichtung beweglich verbunden ist, wobei der zweite Bügel relativ zu der Brücke bewegt wird, wenn die Nahteinrichtung aus ihrer ersten Zwischenanordnung in ihre zweite Zwischenanordnung übergeht.

17. System nach Anspruch 16, **dadurch gekennzeichnet, dass** die Brücke (1023) mit Mitteln (1023C, 1023D) zum axialen Annähern der zwei Bügel (1021, 1022) der oder jeder Nahteinrichtung (1020) versehen ist, wobei diese Annäherungsmittel angepasst sind, um, wenn die Nahteinrichtung in ihrer ausgefahrenen Anordnung ist, den einen der zwei Bügel zu dem anderen hin in axialer Richtung zu führen, insbesondere an die Stelle, wo sie die Wände des Gangs durchqueren, wobei gleichzeitig die zwei Bügel einander gegenüber zurückgehalten werden, um zu verhindern, dass sie sich in axialer Richtung beabstanden, derart, dass die Anastomose zwischen den Gängen gequetscht wird.

18. System nach einem der Ansprüche 1 bis 15, **dadurch gekennzeichnet, dass** die zwei Bügel (21, 22) der oder jeder Nahteinrichtung (20), entgegengesetzt zu ihrem freien Ende (21A, 22A), dauerhaft elastisch verformbar miteinander verbunden sind, so dass die Nahteinrichtung ein, insbesondere einstückiges, Nahtelement (20) bildet.

## Claims

1. A surgical treatment system for performing an anastomosis between two hollow ducts (2, 3) of a patient, such as the bladder (2) and urethra (3) of the patient, in particular after a prostatectomy, said system comprising:
- at least one suture member (20; 1020), made from a bioresorbable material and including two legs (21, 22; 1021, 1022), which have respective free ends (21A, 22A; 1021A, 1022A) and which, outside said free ends, are at least partially deformable,
- an elongated support element (10, 1010), which is suitable both for movably supporting the suture member(s) (20; 1020) and for being inserted along the direction of its central longitudinal axis (X-X) into one of the two hollow ducts (2, 3) of the patient, and
- a mechanism (30; 1030) for biasing the suture member(s) (20; 1020), which is movably supported by the support element (10; 1010), while being at least partially arranged in the support element, and which is suitable for displacing and deforming the or each suture member between a retracted configuration, in which the respective free ends (21A, 22A; 1021A, 1022A) of the two legs (21, 22) of the suture member are movable relative to one another, while each being arranged at a radial distance (δ) from the axis (X-X) that is smaller than the outer radius (r₁₁) of the support element, and a deployed configuration, in which the respective free ends of the two legs of the suture member are arranged outside the support element while each being situated at a radial distance (Δ) from the axis that is larger than the outer radius of the support member, **characterized in that** the respective free ends (21A, 22A; 1021A, 1022A) of the two legs (21, 22; 1021, 1022) are suitable for being fixedly attached to each other by cooperating in a formfitting manner either with each other, or with an attached part (1024) of the suture member, and **in that**, in the deployed configuration, the respective free ends of the two legs are arranged to be fixedly attached to each other, after having transversely passed through the respective walls of the two hollow ducts (2, 3) of the patient from the inside out.

2. The system according to claim 1, **characterized in that** the free end (1021A; 1022A) of one or each of the two legs (1021; 1022) of the or each suture member (1020) has, opposite the rest of the leg, a tip (1021D; 1022D), advantageously capable of perforating the wall of the hollow ducts (2, 3), said tip having, in its connection area with the rest of the leg, a transverse section larger than that of the rest of the leg so as to form a transition shoulder (1021E; 1022E) that is capable of preventing the release of the free end of the leg with respect to said wall, as well as with respect to said attached part (1024) once that free end is engaged with said attached part.

3. The system according to one of claims 1 or 2, **characterized in that** the system also comprises an applicator (40; 1040) for placing and attaching the support element (10) in one of hollow ducts (2, 3).

4. The system according to one of the preceding claims, **characterized in that** the mechanism (1030) and/or the applicator (40) are provided with means (1031B; 1032A; 42) for weakening or even perforating the wall of said hollow duct (2, 3) in one or more portions designed to be passed through by the suture member(s) (20) when they go from their retracted configuration to their deployed configuration.

5. The system according to any one of the preceding claims, **characterized in that** the support element (10) is provided with means (14) for removable immobilization in one of the hollow ducts (2, 3), said immobilizing means in particular being controlled by the applicator (40).

6. The system according to one of claims 3 to 5, **characterized in that** the applicator (1040) has a distal end (1040A) configured to cooperate with and thus command at least part of the mechanism (1030), for example by means of at least one transmission cable.

7. The system according to any one of the preceding claims, **characterized in that** the system also comprises a probe (50; 1050) for commanding the mechanism (30; 1030).

8. The system according to claim 7, **characterized in that** the probe (50; 1050) is suitable for being inserted into one of the ducts (2, 3), in particular the urethra (3) from the meatus to the terminal orifice (3A) of the urethra, and has a distal end (50A; 1050A) configured to cooperate with and thus command at least part of the mechanism (30; 1030).

9. The system according to any one of the preceding claims, **characterized in that** the mechanism (30; 1030) is suitable for displacing and deforming the or each suture member (20; 1020) between the retracted and deployed configurations by successively going through both of two intermediate configurations, i.e., a first intermediate configuration, in which a first (21; 1021) of the two legs (21, 22; 1021, 1022) of the suture member extends through the wall of one of the two hollow ducts (2, 3) of the patient, with its free end (21A; 1021A) situated outside that wall and not fixedly attached to the free end (22A; 1022A) of the second leg (22; 1022), and a second intermediate configuration, in which the second leg (22; 1022) extends through the wall of the other of the two hollow ducts, with its free end (22A; 1022A) situated outside that wall and not fixedly attached to the free end (21A; 1021A) of the first leg (21; 1021).

10. The system according to claim 9, **characterized in that** the mechanism (30) includes:
- a lifting strip (31) which, relative to the support element (10), is translated we mounted along the longitudinal axis (X-X) and is capable of translating the or each suture member (20) to move it from its retracted configuration to its first intermediate configurations;
- a push-piece (32), which is translatably mounted along the axis (X-X) relative to the lifting strip (31) and which is suitable for deforming, in particular by ramp effect, the second leg (22) of the or each suture member (20) to move that suture member from its first intermediate configuration to its second intermediate configuration, and
- a guide (33), which is fixedly attached to the support element (10) along the axis (X-X) and which, for the or each suture member (20), includes a separating portion (332) inserted between the two legs (21, 22) of the suture member and delimiting both a ramp surface (332B) for the first leg (21) and a radial immobilization surface (332A) for the second leg (22) such that, by translating (F2) the lifting strip (31), the free end (21A) of the first leg (21) moves away, in a direction substantially radial to the axis, with respect to the free end (22A) of the second leg (22) when the suture member goes from its retracted configuration to its first intermediate configuration, then, by opposite translation (F3) of the push-piece (32), the free end (22A) of the second leg (22) comes closer, along a direction substantially radial to the axis, with respect to the free end (21A) of the first leg (21) when the suture member goes from its first intermediate configuration to its second intermediate configuration.

11. The system according to claim 10, **characterized in that** the guide (33) is rotatably mounted on the axis (X-X) relative to the support element (10) and is suitable, while the or each suture member (20) is in its second intermediate configuration, for being rotated around itself so as to release, along a direction orthoradial to the axis, its separating portion (332) between the two legs (21, 22) of the suture member to move that suture member into its deployed configuration.

12. The system according to claim 11, **characterized in that**, for the or each suture member (20), the separating portion (332) of the guide (33) is provided with the heel (334), which extends, along a direction peripheral to the axis (X-X), the radial immobilization surface (332A) for the second leg (22) and which is connected to the ramp surface (332B) by a hollow shoulder (335) in which the first leg (21) is received by elastic return to mechanically engage the free end (21A) of said first leg with the free end (22A) of the second leg (22).

13. The system according to any one of claims 10 to 12, **characterized in that** the lifting strip (31) is rotatably mounted around the axis (X-X) relative to the support element (10) and is suitable, while the or each suture member (20) goes from its second intermediate configuration to its deployed configuration, for radially displacing, by cam effect, the end (21B) of the first leg (21) of the suture member, opposite its free end (21A), radially toward the outside of the support element.

14. The system according to claim 9, **characterized in that** the mechanism (1030) includes:
- for each suture member (1020), an arm (1031) that is tiltably mounted on the support element (1010) and along at least part of which the first leg (1021) of the suture member (1020) extends such that the arm stresses and guides the displacement of said first leg to move the suture member from its retracted configuration to its first intermediate configuration; and
- at least one push-piece (1032), which is translatably mounted along the axis (X-X) relative to the support element (1010) and which is suitable for acting on the second leg (1022) of the suture member(s) (1020) and stressing it to deform relative to the rest of the suture member, in particular by ramp effect, to move each suture member from its first intermediate configuration to its second intermediate configuration.

15. The system according to any one of claims 9 to 14, **characterized in that** the first leg (1021) of the or each suture member (1020) extends along a part of the mechanism (1030), in particular along two segments (1031A, 1031B) included by the arm (1031) and which are articulated to each other, such that, in particular by relative tilting of the two aforementioned segments, the first leg of the suture member in its first intermediate configuration successively has a general U shape and a general L shape to tubularize the wall of the hollow duct (2) through which the first leg extends.

16. The system according to any one of the preceding claims, **characterized in that** the or each suture member (1020) also includes a movable connecting bridge (1023) between the two legs (1021, 1022), to which bridge one and/or the other of the two legs are movably assembled, in particular to which bridge the first leg (1021) of the suture member is movably assembled, the first leg being moved relative to the bridge when the suture member goes from its retracted configuration to its first intermediate configuration, and/or to which bridge the second leg (1022) of the suture member is movably assembled, the second leg being moved relative to the bridge when the suture member goes from its first intermediate configuration to its second intermediate configuration.

17. The system according to claim 16, **characterized in that** the bridge (1023) is provided with means (1023C, 1023D) for axially moving the two legs (1021, 1022) of the or each suture member (1020) closer together, said approach means being suitable so as, when the suture member is in its deployed configuration, to axially guide the two legs toward one another, in particular at the location where they cross the walls of the ducts, while retaining the legs relative to one another to prevent them from separating axially, so as to tighten the anastomosis between the ducts.

18. The system according to any one of claims 1 to 15, **characterized in that** the two legs (21, 22) of the or each suture member (20) are, opposite their free end (21A, 22A), permanently connected to each other elastically deformably such that the suture member forms a suture element (20), in particular in a single piece.
